# EUROPEAN PATENT APPLICATION

(11) **EP 4 796 537 A1**
(43) Date of publication of application: **26.08.2026**
(21) Application number: 24879678.1
(22) Date of filing: 11.10.2024
(51) Int. Cl.: C07D 403/04, C07D 401/14, C07D 403/14, C07D 407/14, C07D 409/14, C07D 413/10, C07D 413/14, C07D 417/14, C07D 498/04, H10K 50/858

(54) **CARBAZOLE COMPOUND AND ORGANIC ELECTROLUMINESCENT ELEMENT**

(30) Priority: 17.10.2023 JP 2023179181
(71) Applicant: Hodogaya Chemical Co., Ltd., Tokyo, 105-0021 (JP)
(72) Inventor: KASE, Kouki, Tokyo 105-0021 (JP); YANG, Byung-Sun, Tokyo 105-0021 (JP); CHA, Hyun-Wook, Tokyo 105-0021 (JP); KIM, Hee-Jae, Tokyo 105-0021 (JP); HIRAYAMA, Yuta, Tokyo 105-0021 (JP); HAYASHI, Shuichi, Tokyo 105-0021 (JP)
(74) Representative: Forstmeyer, Dietmar
(86) International application number: PCT/JP2024/036523
(87) International publication number: WO 2025/084253

(57) **Abstract**

It is an object of the present invention to provide a compound that is used to form a capping layer with a high refractive index and a low extinction coefficient at 450 to 750 nm, in order to improve the light extraction efficiency of an organic EL element. The present invention is directed to a compound in which a carbazole skeleton has a quinoxalyl group or a quinazolyl group as a substituent, obtained through molecular designing focusing on the fact that particular carbazole compounds have excellent stability in a thin film state and excellent durability and that the refractive index can be improved by adjusting the molecular structure. By using the compound of the present invention as a material for forming a capping layer, an organic EL element with excellent light emitting efficiency is obtained.

## Description

### Technical field

The present invention relates to a compound suitable for a self-luminescent electronic element favorably used in various display devices, particularly relates to a carbazole compound suitable for an organic electroluminescent element (hereinafter abbreviated as an "organic EL element"), and an organic EL element, an electronic element, and an electronic device using the compound.

### Background Art

Since organic EL elements are self-luminescent elements, they are brighter, have superior display viewability, and can provide a clearer display, compared with liquid crystal elements. For these reasons, active studies have been carried out on organic EL elements.

In 1987, C. W. Tang et al. of Eastman Kodak Company produced a practical organic EL element made of an organic material, by developing an element having a stacked layer structure in which various functions were assigned to different materials. They achieved a high luminance of 1,000 cd/m² or higher at a voltage of 10 V or less by stacking a layer of a fluorescent body capable of transporting electrons and a layer of an organic substance capable of transporting holes, injecting both charges into the fluorescent body layer, and thereby causing the layer to emit light (see Patent Literature 1 and Patent Literature 2, for example).

Many improvements have been heretofore made to organic EL elements to put them to practical use, and high efficiency and high durability are being achieved by forming light emitting elements with a bottom-emission structure that emits light from the bottom, with the functions of various layers in the stacked layer structure being subdivided even further, and an anode, a hole injection layer, a hole transport layer, a light emitting layer, an electron transport layer, an electron injection layer, and a cathode being sequentially provided on a substrate (see Non-Patent Literature 1, for example).

Recently, light emitting elements with a top-emission structure that emits light from the top, with a metal having a high work function being used for an anode, are coming into use. Although light emitting elements with a bottom-emission structure that extracts light from the bottom having a pixel circuit have an issue in that the area of the light emitting portion is limited, light emitting elements with a top-emission structure are advantageous in that a large light emitting portion can be realized because the pixel circuit does not interfere with extraction of light from the top. In light emitting elements with a top-emission structure, translucent electrodes made of LiF/Al/Ag (see Non-Patent Literature 2, for example), Ca/Mg (see Non-Patent Literature 3, for example), LiF/MgAg, or the like are used for a cathode.

In such light emitting elements, when light that is emitted by a light emitting layer and incident on another film is incident at a certain angle or more, the light is totally reflected at an interface between the light emitting layer and the other film. Thus, light that can be used has been limited to only a part of the emitted light. Recently, light emitting elements have been proposed in which a "capping layer" with a high refractive index is provided on the outside of a translucent electrode with a low refractive index, in order to improve the light extraction efficiency (see Non-Patent Literature 2 and Non-Patent Literature 3, for example).

Regarding the effect of the capping layer in light emitting elements with a top-emission structure, while a light emitting element using Ir(ppy)₃ as a light emitting material has a current efficiency of 38 cd/A in the case of not having a capping layer, the light emitting element has a current efficiency of 64 cd/A in the case of using a ZnSe film with a thickness of 60 nm as a capping layer, which indicates that the efficiency is improved about 1.7 times. Furthermore, it is indicated that a maximum point of the transmittance and a maximum point of the efficiency of the translucent electrode and the capping layer do not absolutely match each other, and the maximum point of the light extraction efficiency is determined by interference effects (see Non-Patent Literature 3, for example).

Conventionally, it has been proposed to use a fine metal mask to form a capping layer, but this configuration is problematic in that a metal mask is deformed by heat when used at a high temperature, which deteriorates the positioning precision. For example, ZnSe has a high melting point of 1100°C or higher, and thus vapor deposition cannot be performed at an accurate position with a fine metal mask, which may adversely affect the light emitting element (see Non-Patent Literature 3, for example). Furthermore, film forming through sputtering also adversely affects the light emitting element, and thus a capping layer using an inorganic material as a constituent material cannot be suitably used.

In addition, it is also proposed to use tris(8-hydroxyquinoline)aluminum (hereinafter abbreviated as "Alq₃") as a capping layer for adjusting the refractive index (see Non-Patent Literature 2, for example). However, Alq₃, which is known as an organic EL material that is commonly used as a green light emitting material or an electron transport material, has small absorption at around 450 nm, which is close to the light-emitting wavelength of blue light emitting elements, and thus, when it is used for blue light emitting elements, there is a problem in that both the color purity and the light extraction efficiency deteriorate.

In order to improve the element characteristics of an organic EL element and significantly improve the light extraction efficiency thereof, there is a demand for a material for a capping layer that has a high refractive index and a low extinction coefficient, is stable in a thin film state, and has excellent durability.

### Citation List

### Patent Literature

Patent Literature 1: U.S. Patent No. 5792557
Patent Literature 2: U.S. Patent No. 5639914
Patent Literature 3: WO 2014/009310
Patent Literature 4: Korean Patent No. 10-2164767
Patent Literature 5: WO 2023/048118

### Non-Patent Literature

Non-Patent Literature 1: Proceedings of the 9th Meeting of the Japan Society of Applied Physics, 2001, p. 55-61
Non-Patent Literature 2: Appl. Phys. Lett., (U.S.), 2001, Vol. 78, p. 544-546
Non-Patent Literature 3: Appl. Phys. Lett., (U.S.), 2003, Vol. 82, p. 466-468
Non-Patent Literature 4: Tetrahedron (Netherlands), 2002, Vol. 58, 9633-9695
Non-Patent Literature 5: Appl. Phys. Lett., (U.S.), 2011, Vol. 98, p. 083302

### Summary of Invention

It is an object of the present invention to provide a compound that has a high refractive index and a low extinction coefficient in a wavelength range of 450 to 750 nm, which is favorably used as a material for a capping layer of an organic EL element. Also, it is another object of the invention to provide an organic EL element whose light extraction efficiency has been improved by using the compound.

A compound suitable for a capping layer of an organic EL element is required to have the following physical properties: (1) a high refractive index, (2) a low extinction coefficient, (3) vapor-depositability, (4) good stability in a thin film state, and (5) a high glass transition point. Also, an organic EL element that is to be provided by the present invention is required to have the following physical properties: (1) high light extraction efficiency, (2) no deterioration in the color purity, (3) light transmittability without change over time, and (4) a long life.

To achieve the above-described objects, the inventors of the present invention optimized the molecular design focusing on the fact that a carbazole compound has excellent stability in a thin film state and excellent durability, and thus developed a material that has a high refractive index and a low extinction coefficient in a wavelength range of 450 to 750 nm. Furthermore, the inventors produced an organic EL element using the compound and thoroughly evaluated the properties thereof, as a result of which it was found that the conventional problems are solved, and the present invention was accomplished.

That is to say, the present invention provides a carbazole compound represented by a general formula (I) below and an organic EL element using the same.
1) A carbazole compound represented by a general formula (I) below. 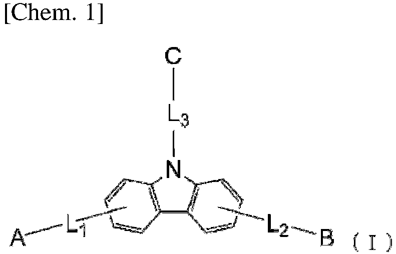 In the formula, A, B, and C each independently represent a substituted or unsubstituted monovalent aryl group or a substituted or unsubstituted monovalent heteroaryl group.
   C is not a benzooxazolyl group, and at least two of A, B, and C represent a substituted or unsubstituted quinoxalyl group or a substituted or unsubstituted quinazolyl group.
   L₁ to L₃ each independently represent a single bond, an unsubstituted divalent aryl group, or an unsubstituted divalent heteroaryl group.
2) The carbazole compound as set forth in 1), represented by a general formula (II) below. 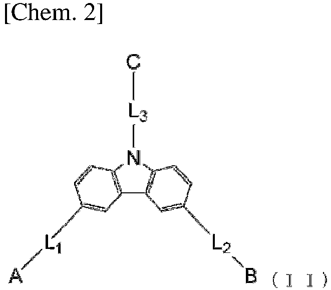 In the formula, A, B, and C and L₁ to L₃ are as defined in the general formula (I) above.
3) The carbazole compound as set forth in 2), wherein in the general formula (II) above, L₁ to L₃ are each independently a single bond, an unsubstituted phenylene group, an unsubstituted pyridylene group, or an unsubstituted naphthylene group.
4) The carbazole compound as set forth in 3), wherein in the general formula (II) above, A and B or A and C are each independently a substituted or unsubstituted quinoxalyl group or a substituted or unsubstituted quinazolyl group.
5) The carbazole compound as set forth in 4),
   wherein in the general formula (II) above, L₁ and L₂ are a single bond, and
   A and B are each independently a substituted quinoxalyl group or a substituted quinazolyl group.
6) The carbazole compound as set forth in 5), wherein in the general formula (II) above, A and B are each independently a substituted 6-quinoxalyl group or a substituted 6-quinazolyl group.
7) The carbazole compound as set forth in 4),
   wherein in the general formula (II) above, L₁ and L₂ are a single bond, and
   A and B are each independently an unsubstituted quinoxalyl group or an unsubstituted quinazolyl group, or
   in the general formula (II) above, L₁ and L₃ are a single bond, and
   A and C are each independently an unsubstituted quinoxalyl group or an unsubstituted quinazolyl group.
8) The carbazole compound as set forth in 7), wherein in the general formula (II) above, A and B are each independently an unsubstituted 2-quinoxalyl group or an unsubstituted 2-quinazolyl group.
9) The carbazole compound as set forth in 4),
   wherein in the general formula (II) above, L₁ and L₂ are each independently an unsubstituted phenylene group, an unsubstituted pyridylene group, or an unsubstituted naphthylene group, and
   A and B are each independently an unsubstituted quinoxalyl group or an unsubstituted quinazolyl group.
10) The carbazole compound as set forth in 7), wherein in the general formula (II) above, A and B are each independently an unsubstituted 2-quinoxalyl group or an unsubstituted 2-quinazolyl group.
11) The carbazole compound as set forth in 2), wherein in the general formula (II) above, C is a naphthyl group, a cyanophenyl group, a pyridyl group, a quinolyl group, a quinoxalyl group, a quinazolyl group, a benzothiazolyl group, or an oxazolopyridyl group.
12) The carbazole compound as set forth in 11), wherein in the general formula (II) above, C is a 2-naphthyl group, a 4-cyanophenyl group, a 3-pyridyl group, a 4-pyridyl group, a 2-quinolyl group, a 3-quinolyl group, a 6-quinolyl group, a 7-quinolyl group, a 2-quinoxalyl group, a 6-quinoxalyl group, a 2-quinazolyl group, a 6-quinazolyl group, a 7-quinazolyl group, a 2-benzothiazolyl group, or a 2-oxazolopyridyl group.
13) An organic EL element at least comprising an anode electrode, a hole transport layer, a light emitting layer, an electron transport layer, a cathode electrode, and a capping layer arranged in this order, wherein the capping layer contains the carbazole compound as set forth in any one of 1) to 12).
14) The organic EL element as set forth in 13), wherein a vapor-deposited film, which is obtained by subjecting the carbazole compound as set forth in any one of 1) to 12) vacuum vapor deposition to form a film having a thickness of 80 nm on a silicon substrate, has a refractive index of 1.70 or more at a wavelength of 450 nm or more and 750 nm or less as measured at room temperature (25±2°C).
15) The organic EL element as set forth in 13), wherein the capping layer is constituted by stacked layers or a mixed layer containing two or more types of compounds, and at least one of the compounds is the carbazole compound as set forth in any one of 1) to 12).
16) An electronic device or an electronic element comprising a pair of electrodes and at least one organic layer held therebetween, wherein the electronic device or the electronic element contains the carbazole compound as set forth in any one of 1) to 12).

### Advantageous Effects of Invention

Since the carbazole compound represented by the general formula (I) of the present invention has (1) a high refractive index in a wavelength range of 450 nm or more and 750 nm or less, (2) a low extinction coefficient, (3) vapor-depositability, (4) good stability in a thin film state, and (5) a high thermal resistance, it is possible to significantly improve light extraction efficiency by providing a capping layer using such a compound, outside the transparent or translucent electrode of the organic EL element.

### Brief Description of Drawings

[Fig. 1] Fig. 1 shows the structures of Compounds 1 to 15 as examples of the carbazole compound represented by the general formula (I) of the present invention.
[Fig. 2] Fig. 2 shows the structures of Compounds 15 to 27 as examples of the carbazole compound represented by the general formula (I) of the present invention.
[Fig. 3] Fig. 3 shows the structures of Compounds 28 to 39 as examples of the carbazole compound represented by the general formula (I) of the present invention.
[Fig. 4] Fig. 4 shows the structures of Compounds 40 to 48 as examples of the carbazole compound represented by the general formula (I) of the present invention.
[Fig. 5] Fig. 5 shows the structures of Compounds 49 to 60 as examples of the carbazole compound represented by the general formula (I) of the present invention.
[Fig. 6] Fig. 6 shows the structures of Compounds 61 to 75 as examples of the carbazole compound represented by the general formula (I) of the present invention.
[Fig. 7] Fig. 7 shows the structures of Compounds 76 to 87 as examples of the carbazole compound represented by the general formula (I) of the present invention.
[Fig. 8] Fig. 8 shows the structures of Compounds 88 to 97 as examples of the carbazole compound represented by the general formula (I) of the present invention.

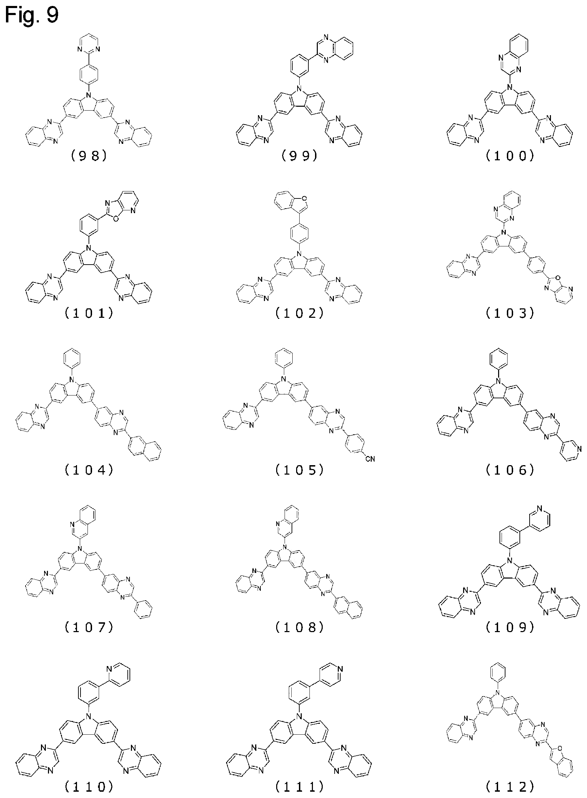

[Fig. 9] Fig. 9 shows the structures of Compounds 98 to 112 as examples of the carbazole compound represented by the general formula (I) of the present invention.
[Fig. 10] Fig. 10 shows an example of the configuration of the organic EL element of the present invention.

### Description of Embodiment

The compound of the present invention is a compound represented by the general formula (I) above.

The aromatic ring constituting the "monovalent aryl group" of the "substituted or unsubstituted monovalent aryl group" represented by A, B, and C in the general formula (I) may be a monocyclic ring, a fused ring constituted by two or more fused rings, a linked ring constituted by two or more rings linked via a single bond, or a spiro ring constituted by two or more rings linked via a spiro bond. If it is a fused ring, the number of rings fused is preferably 2 to 6, and is, for example, 2 to 4. If it is a linked ring, the number of rings linked is preferably 2 to 6, and is, for example, 2 to 4. The number of carbon atoms in the aromatic ring is, for example, 6 to 30, for example, 6 to 22, for example, 6 to 18, for example, 6 to 14, and, for example, 6 to 10. Specific examples of the "monovalent aromatic hydrocarbon group" in A, B, and C may include aryl groups with 6 to 30 carbon atoms, such as a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a fluorenyl group, and a spirobifluorenyl group.

The aromatic heterocyclic ring constituting the "monovalent heteroaryl group" of the "substituted or unsubstituted monovalent heteroaryl group" represented by A, B, and C in the general formula (I) may be a monocyclic ring or a fused ring constituted by two or more fused rings. If it is a fused ring, the number of rings fused is preferably 2 to 6, and is, for example, 2 to 4. Examples of heteroatoms constituting the aromatic heterocyclic ring may include a nitrogen atom, an oxygen atom, and a sulfur atom. The number of carbon atoms in the aromatic heterocyclic ring is, for example, 2 to 40, for example, 2 to 30, and, for example, 2 to 18. Specific examples of the "monovalent heteroaryl group" in A, B, and C may include heteroaryl groups with 2 to 20 carbon atoms, such as a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, an imidazopyridyl group, an oxazolopyridyl group, an oxazolopyrazyl group, a quinoxalyl group, a quinazolyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Regarding the "substituted or unsubstituted monovalent fused polycyclic aromatic group", reference can be made to the description and specific examples concerning the case of a fused ring constituted by two or more fused rings in the description of the "substituted or unsubstituted monovalent aryl group" and the "substituted or unsubstituted monovalent heteroaryl group".

The "divalent aryl group" or the "divalent heteroaryl group" in the "substituted or unsubstituted divalent aryl group" or the "substituted or unsubstituted divalent heteroaryl group" represented by L₁ to L₃ in the general formula (I) may be a divalent group obtained by removing one hydrogen atom from the "monovalent aryl group" or the "monovalent heteroaryl group" represented by A, B, and C in the general formula (I), for example, a divalent group obtained by removing one hydrogen atom from the above-listed specific groups.

Specific examples of the "substituent" in the "substituted monovalent aryl group" or the "substituted monovalent heteroaryl group" represented by A, B, and C in the general formula (I) may include:
a heavy hydrogen atom, a cyano group, a nitro group;
halogen atoms such as a fluorine atom, a chlorine atom, a bromine atom, and an iodine atom;
silyl groups such as a trimethylsilyl group and a triphenylsilyl group;
linear or branched alkyl groups with 1 to 6 carbon atoms such as a methyl group, an ethyl group, and a propyl group;
linear or branched alkyloxy groups with 1 to 6 carbon atoms such as a methyloxy group, an ethyloxy group, and a propyloxy group;
alkenyl groups such as a vinyl group and an allyl group;
aryl groups with 6 to 30 carbon atoms such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group;
heteroaryl groups with 2 to 20 carbon atom such as a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzooxazolyl group, a benzothiazolyl group, an imidazopyridyl group, an oxazolopyridyl group, an oxazolopyrazyl group, a quinoxalyl group, a quinazolyl group, a benzimidazolyl group, a pyrazolyl group, a dibenzofuranyl group, a dibenzothienyl group, a naphthyridinyl group, an acridinyl group, a carbolinyl group, and a phenanthrolinyl group;
aryloxy groups such as a phenyloxy group and a tolyloxy group; and aralkyloxy groups such as a benzyloxy group and a phenethyloxy group.

The hydrogen atoms of these substituents may further be substituted with substituents given as examples herein. Preferred substituents include a heavy hydrogen atom, a linear or branched alkyl group with 1 to 6 carbon atoms, and a monovalent aromatic hydrocarbon group with 6 to 20 ring skeleton-constituting atoms. With respect to substituents substituting other substituents, a substituent directly substituting a parent skeleton (an aromatic hydrocarbon group or an aromatic heterocyclic group) may be referred to as a "first substituent", and a substituent substituting the first substituent may be referred to as a "second substituent". If the first substituent includes a benzene ring, the benzene ring may be bonded to the parent skeleton to form a ring structure. If the two or more substituents substitute a benzene ring of the first substituent, adjacent substituents may be bonded to each other to form a ring structure. In this case, the bond between the benzene ring in the first substituent and the parent skeleton and the bond between the second substituents may be a single bond or a bond via a linking group. Examples of the linking group may include a substituted or unsubstituted methylene group, an oxygen atom, and a sulfur atom.

The "substituent" in the "substituted divalent aryl group" or the "substituted divalent heteroaryl group" represented by L₁ to L₃ in the general formula (I) may be the same as those given as examples of the "substituent" in the "substituted aryl group" or the "substituted heteroaryl group" represented by A, B, and C in the general formula (I), and the possible forms may also be the same.

In the compound of the present invention, at least two of A, B, and C in the general formula (I) represent a substituted or unsubstituted quinoxalyl group or a substituted or unsubstituted quinazolyl group, it is preferable that at least two of A, B, and C are a substituted or unsubstituted 2-quinoxalyl group, a substituted or unsubstituted 6-quinoxalyl group, a substituted or unsubstituted 2-quinazolyl group, or a substituted or unsubstituted 6-quinazolyl group, and it is even more preferable that at least two of A, B, and C are an unsubstituted 2-quinoxalyl group, a substituted 6-quinoxalyl group, an unsubstituted 2-quinazolyl group, or a substituted 6-quinazolyl group.

In the compound of the present invention, it is preferable that groups that are not a substituted or unsubstituted quinoxalyl group or a substituted or unsubstituted quinazolyl group in A, B, and C in the general formula (I) are a naphthyl group, a cyanophenyl group, a pyridyl group, a quinolyl group, a benzothiazolyl group, or an oxazolopyridyl group, and it is more preferable that they are a 2-naphthyl group, a 4-cyanophenyl group, a 3-pyridyl group, a 4-pyridyl group, a 4-pyridyl group, a 2-quinolyl group, a 3-quinolyl group, a 6-quinolyl group, a 7-quinolyl group, a 2-quinoxalyl group, a 6-quinoxalyl group, a 2-quinazolyl group, a 6-quinazolyl group, a 7-quinazolyl group, a 2-benzothiazolyl group, or a 2-oxazolopyridyl group.

A, B, and C in the general formula (I) may be any combination selected from the above-listed groups. It is preferable that A and B are a substituted or unsubstituted quinoxalyl group or a substituted or unsubstituted quinazolyl group and C is a group other than these, or that A and C are a substituted or unsubstituted quinoxalyl group or a substituted or unsubstituted quinazolyl group and B is a group other than these.

L₁ to L₃ in the general formula (I) are preferably a single bond, an unsubstituted phenylene group, an unsubstituted pyridylene group, or an unsubstituted naphthylene group, and more preferably a single bond or an unsubstituted phenylene group. L₁ to L₃ may be the same or different from each other.

In the organic EL element, the thickness of the capping layer is preferably in the range of 30 to 120 nm, and more preferably in the range of 40 to 80 nm.

The carbazole compound represented by the general formula (I) above is a novel compound, but can be synthesized, for example, according to known coupling reactions using a palladium catalyst or the like (see Non-Patent Literature 4, for example).

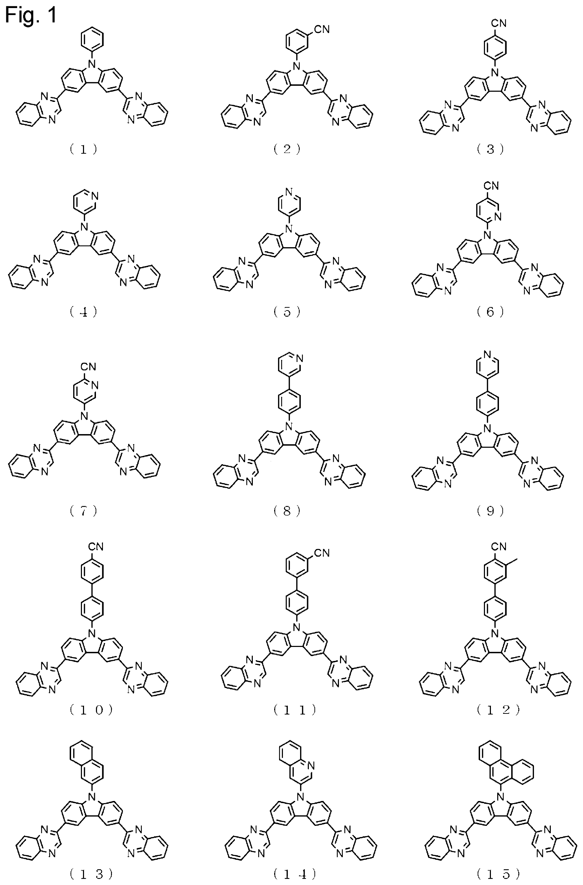

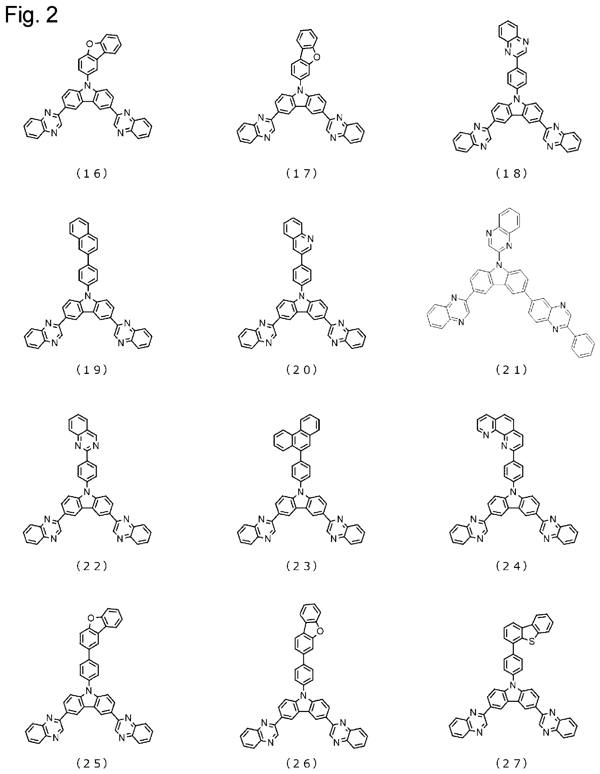

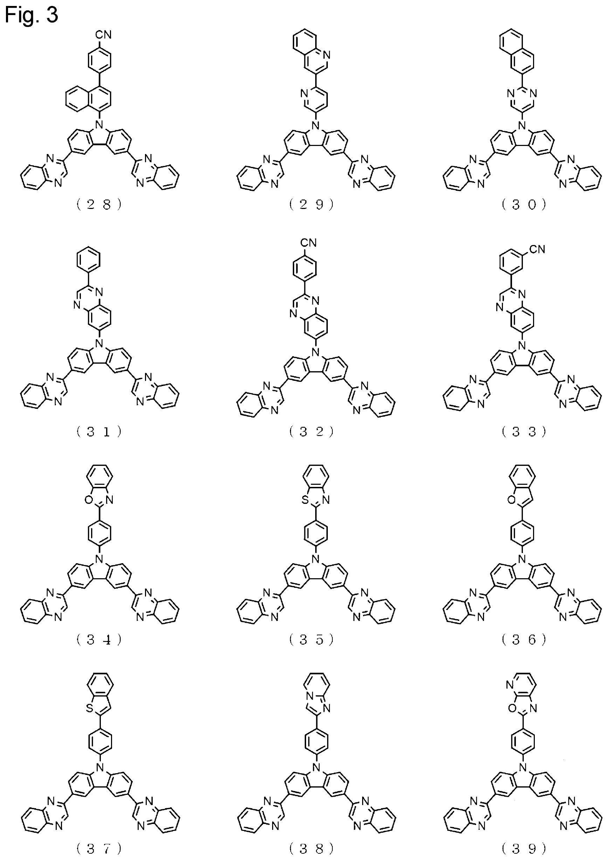

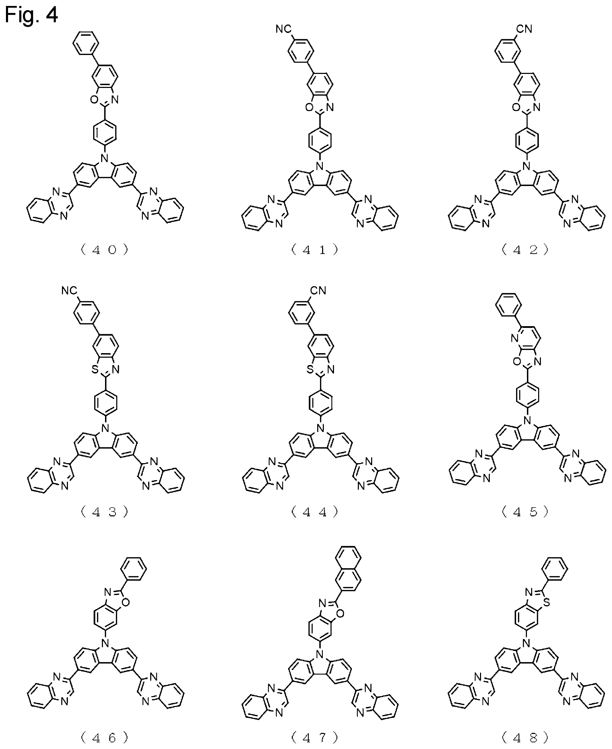

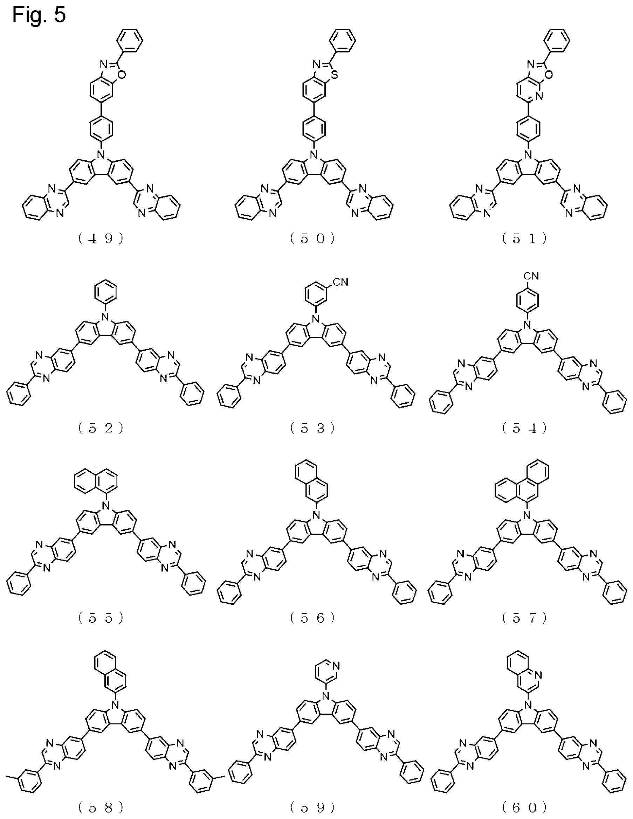

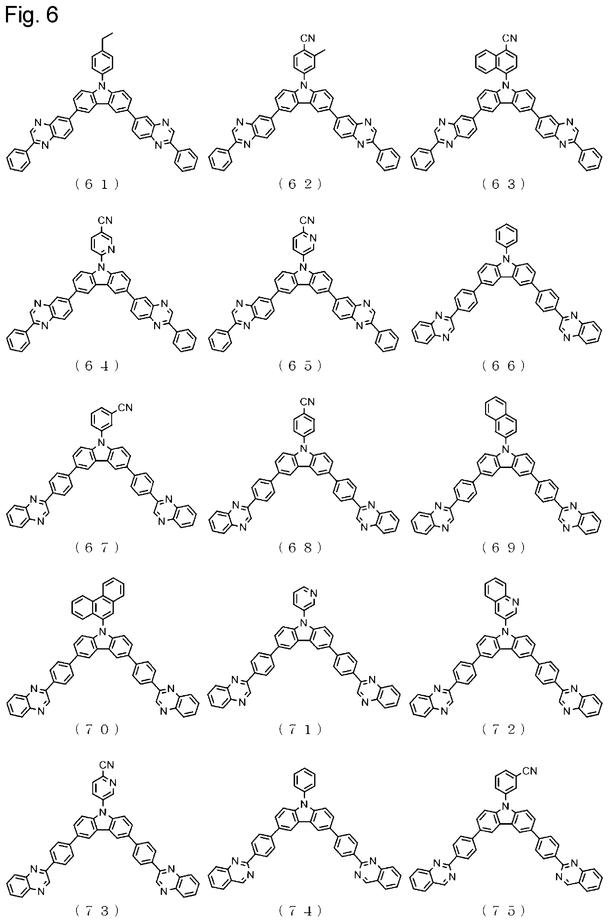

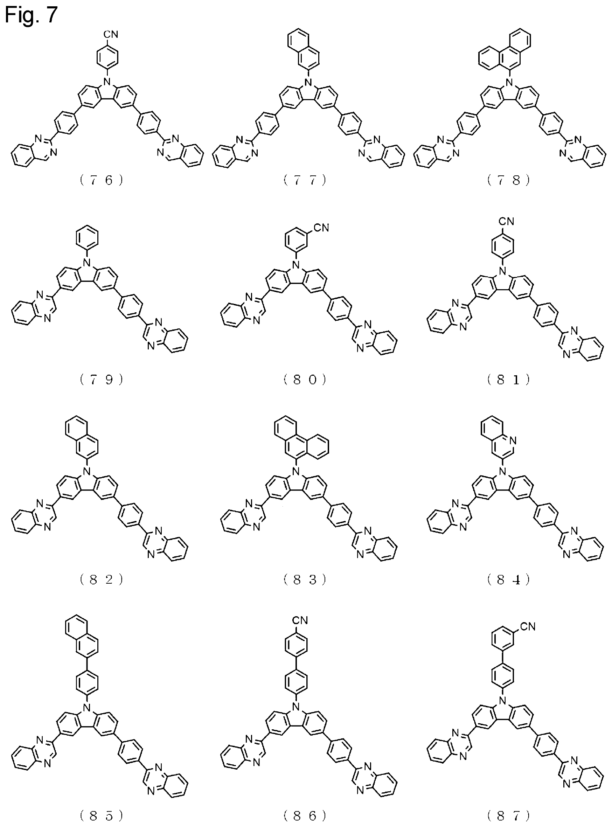

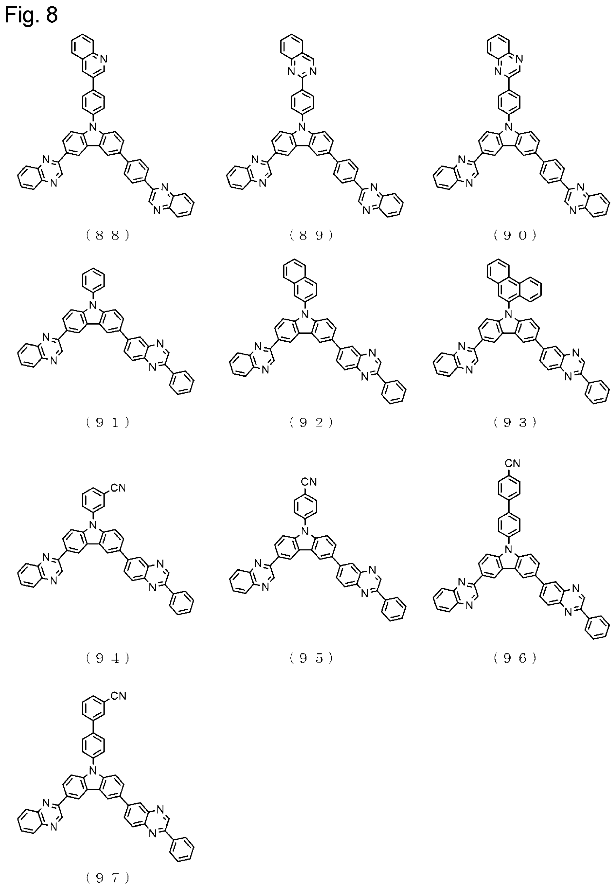

Figs. 1 to 8 show specific examples of preferred carbazole compounds among those represented by the general formula (I) above, but the invention is not limited to these compounds.

These is no particular limitation on the method for purifying the carbazole compound represented by the general formula (I) above, but examples thereof include known methods used for purification of organic compounds, such as purification by column chromatography, adsorption purification using silica gel, activated carbon, activated clay, or the like, recrystallization purification and crystallization purification using solvents, and sublimation purification. Compounds can be identified by NMR analysis.

Furthermore, the melting point, the glass transition point (Tg), the refractive index, and the extinction coefficient are preferably measured as physical properties of the carbazole compound represented by the general formula (I) above. The melting point is an indicator of vapor deposition properties, the glass transition point (Tg) is an indicator of the stability in a thin film state, and the refractive index and extinction coefficient are indicators of the improvement of light extraction efficiency.

The melting point and the glass transition point (Tg) can be obtained by performing measurement on a powder using a high-sensitivity differential scanning calorimeter (DSC3100SA manufactured by Bruker AXS K.K.).

The refractive index and the extinction coefficient can be obtained by performing measurement on an 80-nm thin film formed on a silicon substrate, using a spectroscopic measurement device (F10-RT-UV manufactured by Filmetrics).

The organic EL element for use as a light emitting element with a top-emission structure may have a structure constituted by an anode, a hole transport layer, a light emitting layer, an electron transport layer, a cathode, and a capping layer sequentially provided on a glass substrate. It is also possible to adopt a structure in which a hole injection layer is further provided between the anode and the hole transport layer, a structure in which an electron blocking layer is further provided between the hole transport layer and the light emitting layer, a structure in which a hole blocking layer is further provided between the light emitting layer and the electron transport layer, and a structure in which an electron injection layer is further provided between the electron transport layer and the cathode. In these multilayer structures, a single organic layer may have the functions of a plurality of layers, and, for example, it is possible to adopt a configuration in which an organic layer serves as both the hole injection layer and the hole transport layer, a configuration in which an organic layer serves as both the hole transport layer and the electron blocking layer, a configuration in which an organic layer serves as both the hole blocking layer and the electron transport layer, a configuration in which an organic layer serves as both the electron transport layer and the electron injection layer, and the like. Furthermore, two or more organic layers having the same function may be stacked, and, for example, it is possible to adopt a configuration in which two hole transport layers are stacked, a configuration in which two light emitting layers are stacked, a configuration in which two electron transport layers are stacked, a configuration in which two capping layers are stacked, and the like.

The total film thickness of the layers of the organic EL element is preferably from 200 to 750 nm, and more preferably from 350 to 600 nm. Furthermore, the film thickness of the capping layer is, for example, preferably from 30 to 120 nm, and more preferably from 40 to 80 nm. In this case, it is possible to obtain good light extraction efficiency. Note that the film thickness of the capping layer may be changed as appropriate according to the type of light emitting material used in the light emitting element, the thickness of the organic EL element excluding the capping layer, and the like.

An electrode material having a high work function, such as indium tin oxide (ITO) or gold, is used for the anode of the organic EL element.

Arylamine compounds having a molecular structure in which three or more triphenylamine structures are bonded to each other via a single bond or a divalent group not containing a hetero atom, for example, starburst triphenylamine derivatives, materials such as various triphenylamine tetramers, porphyrin compounds typified by copper phthalocyanine, acceptor type heterocyclic compounds such as hexacyanoazatriphenylene, and coating type polymer materials can be used as a material for the hole injection layer of the organic EL element. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using a known method such as vapor deposition as well as spin coating or inkjet printing.

Benzidine derivatives such as N,N'-diphenyl-N,N'-di(m-tolyl)benzidine (hereinafter abbreviated as "TPD"), N,N'-diphenyl-N,N'-di(α-naphthyl)benzidine, and N,N,N',N'-tetrabiphenylyl benzidine, 1,1-bis[4-(di-4-tolylamino)phenyl]cyclohexane, and the like are used as a material for the hole transport layer of the organic EL element. In particular, it is preferable to use arylamine compounds having a molecular structure in which two triphenylamine structures are bonded to each other via a single bond or a divalent group not containing a hetero atom, for example, N,N,N',N'-tetrabiphenylyl benzidine and the like. It is also preferable to use arylamine compounds having a molecular structure in which three or more triphenylamine structures are bonded to each other via a single bond or a divalent group not containing a hetero atom, for example, various triphenylamine trimers and tetramers. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. Furthermore, coating type polymer materials such as poly(3,4-ethylenedioxythiophene)/poly(styrenesulfonate) can be used for the hole injection and transport layers. With these materials, a thin film can be formed using a known method such as vapor deposition as well as spin coating or inkjet printing.

Furthermore, a material that is obtained by p-doping a material commonly used for the hole injection layer and the hole transport layer with a dopant such as trisbromophenylamine hexachloroantimony, a radialene derivative (see Patent Literature 3, for example), or the like, a polymer compound that has the structure of a benzidine derivative, such as TPD, in a partial structure thereof, and the like can be used as a material for the hole injection layer and the hole transport layer.

Furthermore, it is also possible to stack an electron blocking layer on the organic EL element. Compounds having an electron blocking effect, such as: carbazole derivatives such as 4,4',4"-tri(N-carbazolyl)triphenylamine (hereinafter abbreviated as "TCTA"), 9,9-bis[4-(carbazol-9-yl)phenyl]fluorene, 1,3-bis(carbazol-9-yl)benzene (hereinafter abbreviated as "mCP"), and 2,2-bis(4-carbazol-9-yl-phenyl)adamantane; and compounds that have a triphenylsilyl group and a triarylamine structure and are typified by 9-[4-(carbazol-9-yl)phenyl]-9-[4-(triphenylsilyl)phenyl]-9H-fluorene, can be used as a material for the electron blocking layer. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using a known method such as vapor deposition as well as spin coating or inkjet printing.

In addition to metal complexes of quinolinol derivatives such as Alq₃, various types of metal complexes, an anthracene derivative, a bisstyrylbenzene derivative, a pyrene derivative, an oxazole derivative, a poly(p-phenylene vinylene) derivative, and the like can be used as a material for the light emitting layer of the organic EL element. Moreover, the light emitting layer may also be formed using a host material and a dopant material. As the host material, an anthracene derivative is preferably used, and heterocyclic compounds having an indole ring as a partial structure of a fused ring, heterocyclic compounds having a carbazole ring as a partial structure of a fused ring, a carbazole derivative, a thiazole derivative, a benzimidazole derivative, a polydialkylfluorene derivative, and the like can be used in addition to the above-listed light emitting materials. As the dopant material, quinacridone, coumarin, rubrene, perylene, and derivatives thereof; a benzopyran derivative; a rhodamine derivative; an aminostyryl derivative; and the like can be used, and green light emitting materials are preferably used. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked.

Furthermore, it is also possible to use a phosphorescent emitter as the light emitting material. As the phosphorescent emitter, a phosphorescent emitter of a metal complex of iridium, platinum, or the like can be used. Examples thereof include a green phosphorescent emitter such as Ir(ppy)₃, a blue phosphorescent emitter such as FIrpic or FIr6, a red phosphorescent emitter such as Btp₂Ir(acac), and the like, and it is preferable to use a green phosphorescent emitter. In this case, as host materials having hole injectability and transportability, carbazole derivatives such as 4,4'-di(N-carbazolyl)biphenyl, TCTA, and mCP can be used as the host material. Furthermore, as host materials having electron transportability, p-bis(triphenylsilyl)benzene, 2,2',2"-(1,3,5-phenylene)-tris(1-phenyl-1H-benzimidazole), and the like can be used.

In order to avoid concentration quenching, it is preferable that doping of the host material with a phosphorescent light emitting material is performed within a range of 1 to 30 wt% with respect to the entire light emitting layer through co-deposition.

As the light emitting material, materials that emit delayed fluorescence, such as CDCB derivatives such as PIC-TRZ, CC2TA, PXZ-TRZ, and 4CzIPN, can also be used (see Non-Patent Literature 5, for example). With these materials, a thin film can be formed using a known method such as vapor deposition as well as spin coating or inkjet printing.

Furthermore, it is also possible to stack a hole blocking layer on the organic EL element. Compounds having a hole blocking effect, such as a phenanthroline derivative such as bathocuproine, a metal complex of a quinolinol derivative, such as aluminum(III)bis(2-methyl-8-quinolinato)-4-phenylphenolate (hereinafter abbreviated as BAlq), various types of rare-earth complexes, a triazole derivative, a triazine derivative, a pyrimidine derivative, an oxadiazole derivative, a benzoazole derivative, and the like can be used as a material for the hole blocking layer. These materials may also be used as the material for the electron transport layer. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using a known method such as vapor deposition as well as spin coating or inkjet printing.

In addition to metal complexes of quinolinol derivatives such as Alq₃ and BAlq, various types of metal complexes, a triazole derivative, a triazine derivative, a pyrimidine derivative, an oxadiazole derivative, a pyridine derivative, a benzimidazole derivative, a benzoazole derivative, a thiadiazole derivative, an anthracene derivative, a carbodiimide derivative, a quinoxaline derivative, a pyridoindole derivative, a phenanthroline derivative, a silole derivative, and the like can be used for the electron transport layer of the organic EL element. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using a known method such as vapor deposition as well as spin coating or inkjet printing.

Alkali metal salts such as lithium fluoride and cesium fluoride, alkaline earth metal salts such as magnesium fluoride, metal complexes of quinolinol derivatives such as lithium quinolinol, metal oxides such as aluminum oxide, metals such as ytterbium (Yb), samarium (Sm), calcium (Ca), strontium (Sr), and cesium (Cs), and the like can be used for the electron injection layer of the organic EL element. When an electron transport layer and a cathode are suitably selected, the electron injection layer can be omitted.

Furthermore, a material obtained by n-doping a material commonly used for the electron injection layer and the electron transport layer with a metal such as cesium can be used as a material for the electron injection layer or the electron transport layer.

A metal having a low work function such as aluminum, an alloy having an even lower work function such as a magnesium-silver alloy, a magnesium-calcium alloy, a magnesium-indium alloy, or an aluminum-magnesium alloy, ITO, IZO, or the like is used as a material for the cathode of the organic EL element.

The carbazole compound represented by the general formula (I) above is favorably used for the capping layer of the organic EL element. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using a known method such as vapor deposition as well as spin coating or inkjet printing.

The refractive index of the light in a wavelength range of 450 to 700 nm transmitted through a film formed from the carbazole compound represented by the general formula (I) above is preferably 1.70 or more, and more preferably 1.85 or more.

In the description above, an organic EL element with a top-emission structure has been described, but the present invention is not limited thereto, and can also be applied in a similar manner to an organic EL element with a bottom-emission structure or an organic EL element with a dual-emission structure that emits light from both the top and the bottom. In these cases, an electrode located in a direction in which light is extracted from the light emitting element to the outside is preferably transparent or translucent.

### Examples

Hereinafter, an embodiment of the present invention will be described in greater detail using examples. However, the present invention is not limited to the examples below as long as it is within the gist thereof.

### Example 1

### Synthesis of Compound (3)

4-{3,6-Bis(4,4,5,5-tetramethyl-1,3,2-dioxaborolane-2-yl)-9H-carbazole-9-yl}ben zonitrile: 7.5 g,
2-chloroquinoxaline: 5.0 g,
tetrakis(triphenylphosphine)palladium(0): 1.7 g, and
potassium carbonate: 10.0 g
were added to a reaction vessel, and the mixture was refluxed and stirred in toluene/EtOH/H₂O mixed solvent overnight.

After the mixture was allowed to cool, MeOH/H₂O was added thereto, and the precipitated solid was filtered to obtain a crude product.

The obtained crude product was purified through recrystallization using o-dichlorobenzene solvent to obtain Compound (3): 5.6 g (yield: 74.1%).

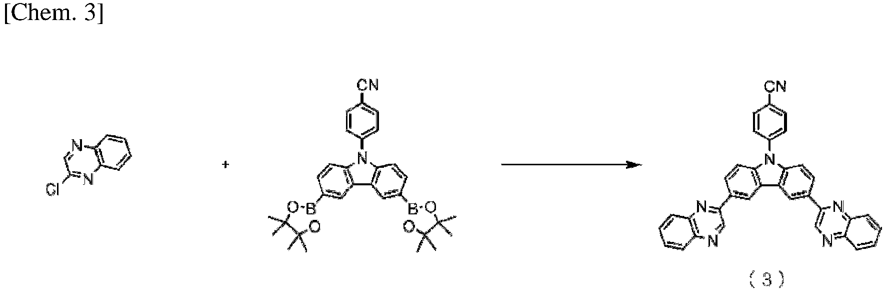

The structure of the obtained yellow powder was identified using NMR.

In ¹H-NMR (CDCl₃), the following signals of 20 hydrogens were detected, and thus it was seen that the obtained material is Compound (3).
δ (ppm) = 9.55 (2H), 9.20 (2H), 8.41 (2H), 8.25 (2H), 8.19 (2H), 8.04 (2H), 7.88-7.78 (6H), 7.66 (2H).

### Example 2

### Synthesis of Compound (13)

9-(Naphthalene-2-yl)-3,6-bis(4,4,5,5-tetramethyl-1,3,2-dioxaborolane-2-yl)-9H-c arbazole: 8.0 g,
2-chloroquinoxaline: 5.1 g,
tetrakis(triphenylphosphine)palladium(0): 1.7 g, and
potassium carbonate: 10.1 g
were added to a reaction vessel, and the mixture was refluxed and stirred in toluene/EtOH/H₂O mixed solvent overnight.

After the mixture was allowed to cool, MeOH/H₂O was added thereto, and the precipitated solid was filtered to obtain a crude product.

The obtained crude product was purified through recrystallization using monochlorobenzene solvent to obtain Compound (13): 6.5 g (yield: 80.4%).

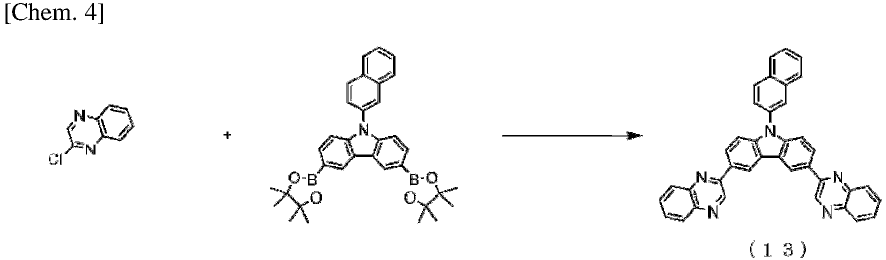

The structure of the obtained yellow powder was identified using NMR.

In ¹H-NMR (CDCl₃), the following signals of 23 hydrogens were detected, and thus it was seen that the obtained material is Compound (13).
δ (ppm) = 9.56 (2H), 9.23 (2H), 8.39 (2H), 8.25 (2H), 8.19-8.17 (4H), 8.07-8.00 (2H), 7.84 (2H), 7.78 (3H), 7.67 (4H).

### Example 3

### Synthesis of Compound (56)

9-(Naphthalene-2-yl)-3,6-bis(4,4,5,5-tetramethyl-1,3,2-dioxaborolane-2-yl)-9H-c arbazole: 5.0 g,
6-chloro-2-phenyl-quinoxaline: 4.6 g,
tris(dibenzylideneacetone)dipalladium(0): 0.4 g,
tricyclohexylphosphine: 0.5 g, and
tripotassium phosphate: 9.7 g
were added to a reaction vessel, and the mixture was refluxed and stirred in 1,4-dioxane/H₂O mixed solvent overnight.

After the mixture was allowed to cool, MeOH/H₂O was added thereto, and the precipitated solid was filtered to obtain a crude product.

The obtained crude product was purified through recrystallization using o-dichlorobenzene solvent to obtain Compound (56): 4.7 g (yield: 73.0%).

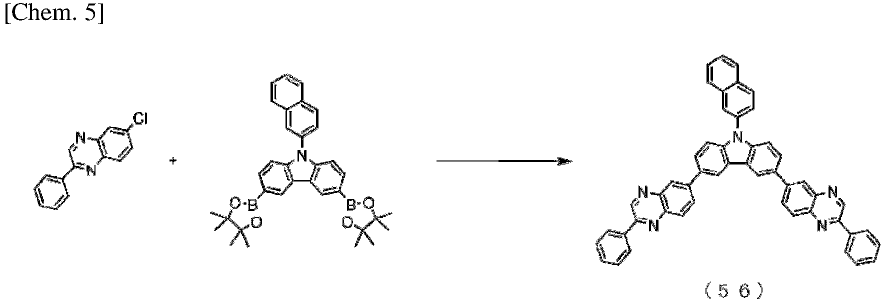

The structure of the obtained yellow powder was identified using NMR.

In ¹H-NMR (CDCl₃), the following signals of 31 hydrogens were detected, and thus it was seen that the obtained material is Compound (56).
δ (ppm) = 9.40 (2H), 8.71 (2H), 8.49 (2H), 8.31 (4H), 8.27 (4H), 8.19-8.17 (2H), 8.05-8.00 (2H), 7.94 (2H), 7.79 (1H), 7.67-7.55 (10H).

### Example 4

### Synthesis of Compound (69)

9-(Naphthalene-2-yl)-3,6-bis(4,4,5,5-tetramethyl-1,3,2-dioxaborolane-2-yl)-9H-c arbazole: 9.0 g,
2-(4-chlorophenyl)-quinoxaline: 8.7 g,
tris(dibenzylideneacetone)dipalladium(0): 0.8 g,
tricyclohexylphosphine: 0.9 g, and
tripotassium phosphate: 10.5 g
were added to a reaction vessel, and the mixture was refluxed and stirred in 1,4-dioxane/H₂O mixed solvent overnight.

After the mixture was allowed to cool, MeOH/H₂O was added thereto, and the precipitated solid was filtered to obtain a crude product.

The obtained crude product was purified through recrystallization using monochlorobenzene solvent to obtain Compound (69): 5.7 g (yield: 49.1%).

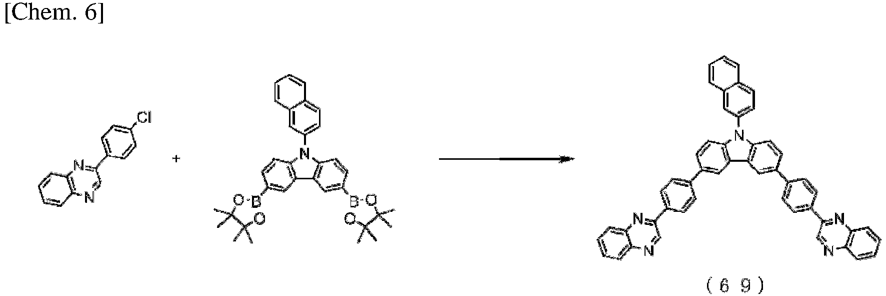

The structure of the obtained yellow powder was identified using NMR.

In ¹H-NMR (CDCl₃), the following signals of 31 hydrogens were detected, and thus it was seen that the obtained material is Compound (69).
δ (ppm) = 9.45 (2H), 8.60 (2H), 8.39 (4H), 8.22 (2H), 8.17 (4H), 8.05-8.00 (6H), 7.86-7.76 (7H), 7.67-7.61 (4H).

### Example 5

### Synthesis of Compound (6)

6-(3,6-Bis(4,4,5,5-tetramethyl-1,3,2-dioxaborolane-2-yl)-9H-carbazole-9-yl)nico tinonitrile: 11.0 g,
2-chloroquinoxaline: 7.6 g,
tetrakis(triphenylphosphine)palladium(0): 1.0 g, and
potassium carbonate: 8.8 g
were added to a reaction vessel, and the mixture was refluxed and stirred in toluene/ethanol/H₂O mixed solvent overnight.

After the mixture was allowed to cool, MeOH was added thereto, and the precipitated solid was filtered to obtain a crude product.

The obtained crude product was purified through recrystallization using dichlorobenzene solvent to obtain Compound (6): 7.5 g (yield: 67.6%).

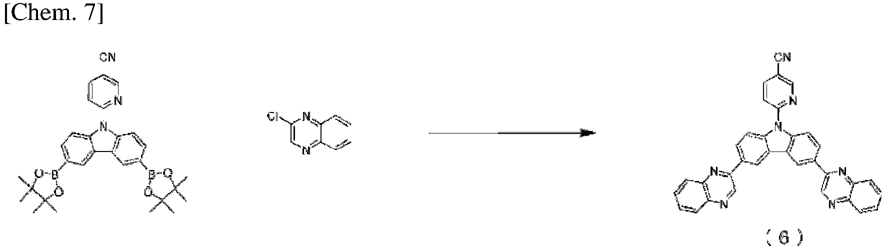

The obtained yellow powder was analyzed using LC-MS, and the same molecular weight was detected.
MS[M+H]⁺= 526

### Example 6

### Synthesis of Compound (10)

3,6-Di(quinoxaline-2-yl)-9H-carbazole: 10.0 g,
4'-bromo-[1,1'-biphenyl]-4-carbonitrile: 7.3 g,
tris(dibenzylideneacetone)dipalladium(0): 0.4 g,
Sphos: 0.4 g, and
tert-butyl sodium: 4.5 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (6): 8.0 g (yield: 56.4%).

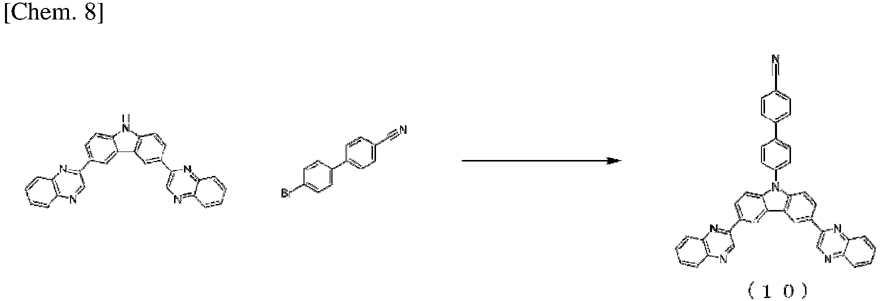

The structure of the obtained yellow powder was identified using NMR.

In ¹H-NMR (CDCl₃), the following signals of 24 hydrogens were detected, and thus it was seen that the obtained material is Compound (10).
δ (ppm) = 9.55 (2H), 9.20 (2H), 8.39 (2H), 8.23 (2H), 8.16 (2H), 7.93 (2H), 7.84-7.75 (10H), 7.67 (2H).

### Example 7

### Synthesis of Compound (17)

3,6-Di(quinoxaline-2-yl)-9H-carbazole: 10.0 g,
3-bromodibenzofuran: 6.4 g,
tris(dibenzylideneacetone)dipalladium(0): 0.4 g,
Sphos: 0.4 g, and
tert-butyl sodium: 4.5 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (17): 11.2 g (yield: 80.6%).

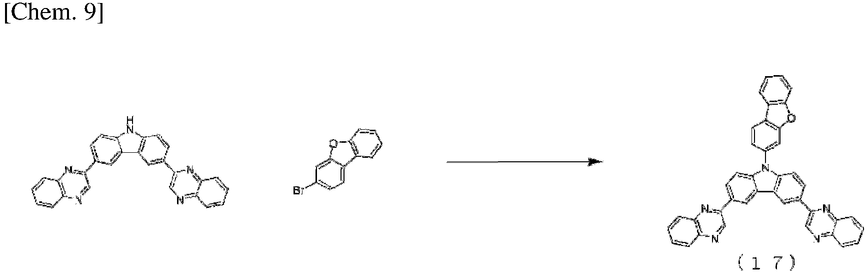

The structure of the obtained yellow powder was identified using NMR.

In ¹H-NMR (CDCl₃), the following signals of 23 hydrogens were detected, and thus it was seen that the obtained material is Compound (17).
δ (ppm) = 9.55 (2H), 9.21 (2H), 8.38 (2H), 8.24 (3H), 8.16 (2H), 8.09 (1H) 7.89 (1H), 7.82 (2H), 7.75 (2H), 7.69-7.64 (3H), 7.57 (1H), 7.46 (1H).

### Example 8

### Synthesis of Compound (18)

3,6-Di(quinoxaline-2-yl)-9H-carbazole: 10.0 g,
2-(4-chlorophenyl)-quinoxaline: 6.3 g,
tris(dibenzylideneacetone)dipalladium(0): 0.5 g,
tri-tert-butylphosphine: 0.2 g, and
tert-butyl sodium: 4.8 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (18): 8.5 g (yield: 57.4%).

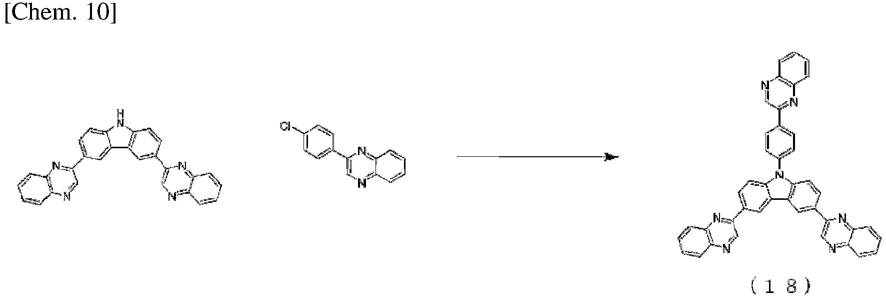

The structure of the obtained yellow powder was identified using NMR.

In ¹H-NMR (CDCl₃), the following signals of 25 hydrogens were detected, and thus it was seen that the obtained material is Compound (18).
δ (ppm) = 9.56 (2H), 9.49 (1H), 9.21 (2H), 8.56 (2H), 8.41 (2H), 8.25-8.15 (6H), 7.92-7.71 (9H).

### Example 9

### Synthesis of Compound (35)

3,6-Di(quinoxaline-2-yl)-9H-carbazole: 5.0 g,
2-(4-bromophenyl)-benzothiazole: 6.0 g,
copper: 2.1 g,
dibenzo-18-crown-6-ether: 0.5 g, and
potassium carbonate: 3.3 g
were added to a reaction vessel, and the mixture was refluxed and stirred in DMF solvent for two nights.

After the mixture was allowed to cool, MeOH/H₂O was added thereto, and the precipitated solid was filtered to obtain a crude product.

The obtained crude product was purified through recrystallization using monochlorobenzene solvent to obtain Compound (35): 4.9 g (yield: 65.6%).

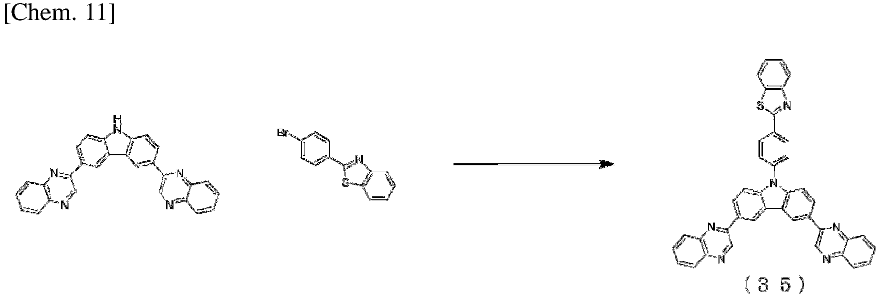

The obtained yellow powder was analyzed using LC-MS, and the same molecular weight was detected.
MS[M+H]⁺= 633

### Example 10

### Synthesis of Compound (36)

3,6-Di(quinoxaline-2-yl)-9H-carbazole: 8.0 g,
2-(4-bromophenyl)-benzofuran: 9.1 g,
copper: 3.3 g,
dibenzo-18-crown-6-ether: 0.6 g, and
potassium carbonate: 5.2 g
were added to a reaction vessel, and the mixture was refluxed and stirred in DMF solvent for three nights.

After the mixture was allowed to cool, MeOH/H₂O was added thereto, and the precipitated solid was filtered to obtain a crude product.

The obtained crude product was purified through recrystallization using dichlorobenzene solvent to obtain Compound (36): 8.6 g (yield: 73.9%).

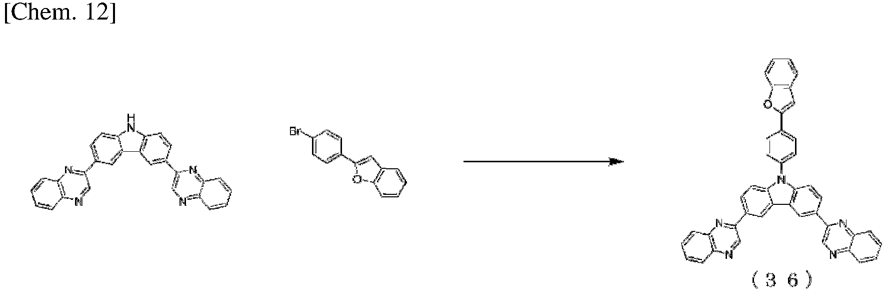

The structure of the obtained yellow powder was identified using NMR.

In ¹H-NMR (CDCl₃), the following signals of 25 hydrogens were detected, and thus it was seen that the obtained material is Compound (36).
δ (ppm) = 9.55 (2H), 9.20 (2H), 8.39 (2H), 8.25-8.15 (6H), 7.84-7.74 (6H), 7.68 (3H), 7.60 (1H), 7.36 (1H), 7.30 (1H), 7.20 (1H).

### Example 11

### Synthesis of Compound (37)

3,6-Di(quinoxaline-2-yl)-9H-carbazole: 8.0 g,
2-(4-bromophenyl)-thiophene: 6.0 g,
tris(dibenzylideneacetone)dipalladium(0): 0.4 g,
tri-tert-butylphosphine: 0.2 g, and
tert-butyl sodium: 2.7 g
were added to a reaction vessel, and the mixture was refluxed and stirred in xylene solvent overnight.

After the mixture was allowed to cool, MeOH/H₂O was added thereto, and the precipitated solid was filtered to obtain a crude product.

The obtained crude product was purified through recrystallization using dichlorobenzene solvent to obtain Compound (37): 9.1 g (yield: 76.2%).

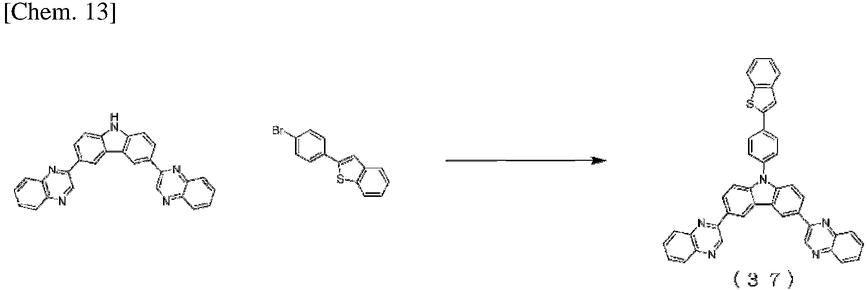

The structure of the obtained yellow powder was identified using NMR.

In ¹H-NMR (CDCl₃), the following signals of 25 hydrogens were detected, and thus it was seen that the obtained material is Compound (37).
δ (ppm) = 9.55 (2H), 9.20 (2H), 8.39 (2H), 8.23 (2H), 8.16 (2H), 8.04 (2H), 7.90-7.67 (11H), 7.40 (2H).

### Example 12

### Synthesis of Compound (39)

3,6-Di(quinoxaline-2-yl)-9H-carbazole: 8.0 g,
2-(4-bromophenyl)-oxazolopyridine: 9.1 g,
copper: 3.3 g,
dibenzo-18-crown-6-ether: 0.6 g, and
potassium carbonate: 5.2 g
were added to a reaction vessel, and the mixture was refluxed and stirred in DMF solvent for two nights.

After the mixture was allowed to cool, MeOH/H₂O was added thereto, and the precipitated solid was filtered to obtain a crude product.

The obtained crude product was purified through recrystallization using dichlorobenzene solvent to obtain Compound (39): 3.0 g (yield: 25.7%).

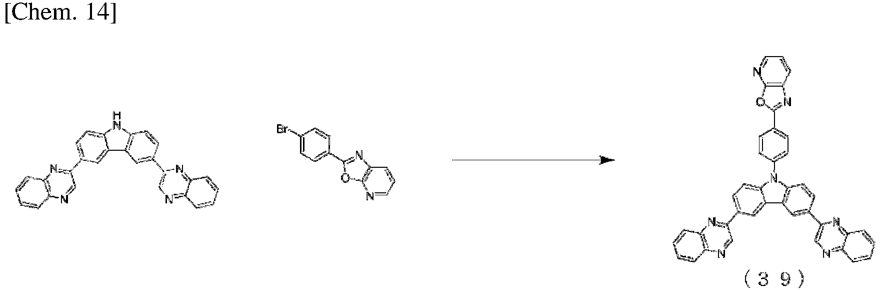

The obtained yellow powder was analyzed using LC-MS, and the same molecular weight was detected.
MS[M+H]⁺= 618

### Example 13

### Synthesis of Compound (54)

(4-(3,6-Dibromo-9H-carbazole-9-yl)benzonitrile: 4.0 g,
2-phenyl-6-(4,4,5,5-tetramethyl-1,3,2-dioxaborolane-2-yl)quinoxaline: 6.9 g, tetrakis(triphenylphosphine)palladium(0): 0.4 g, and
potassium carbonate: 3.9 g
were added to a reaction vessel, and the mixture was refluxed and stirred in toluene/ethanol/H₂O mixed solvent overnight.

After the mixture was allowed to cool, MeOH was added thereto, and the precipitated solid was filtered to obtain a crude product.

The obtained crude product was purified through recrystallization using dichlorobenzene solvent to obtain Compound (54): 4.9 g (yield: 76.6%).

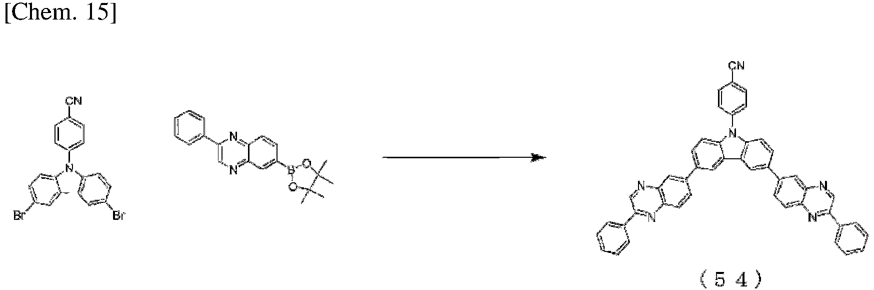

The structure of the obtained yellow powder was identified using NMR.

In ¹H-NMR (CDCl₃), the following signals of 28 hydrogens were detected, and thus it was seen that the obtained material is Compound (54).
δ (ppm) = 9.40 (2H), 8.66 (2H), 8.46 (2H), 8.31-8.24 (8H), 8.01 (2H), 7.94 (2H), 7.86 (2H), 7.65-7.55 (8H).

### Example 14

### Synthesis of Compound (59)

3,6-Dibromo-9-(quinoline-3-yl)-9H-carbazole: 9.0 g,
2-phenyl-6-(4,4,5,5-tetramethyl-1,3,2-dioxaborolane-2-yl)quinoxaline: 9.6 g, tris(dibenzylideneacetone)dipalladium(0): 0.8 g,
tricyclohexylphosphine: 1.0 g, and
tripotassium phosphate: 11.6 g
were added to a reaction vessel, and the mixture was refluxed and stirred in 1,4-dioxane/H₂O mixed solvent overnight.

After the mixture was allowed to cool, MeOH was added thereto, and the precipitated solid was filtered to obtain a crude product.

The obtained crude product was purified through recrystallization using monochlorobenzene/acetone mixed solvent to obtain Compound (59): 8.6 g (yield: 68.2%).

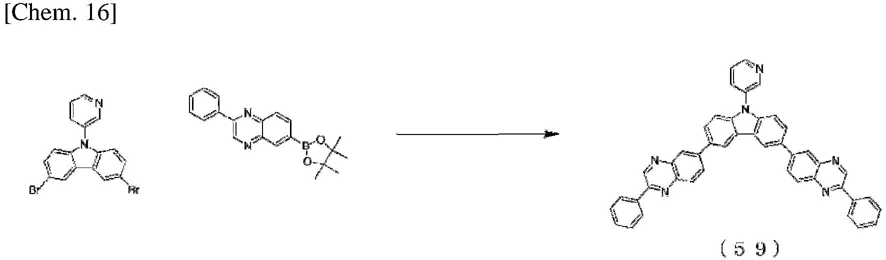

The structure of the obtained yellow powder was identified using NMR.

In ¹H-NMR (CDCl₃), the following signals of 28 hydrogens were detected, and thus it was seen that the obtained material is Compound (59).
δ (ppm) = 9.39 (2H), 9.01 (1H), 8.82 (1H), 8.67 (2H), 8.47 (2H), 8.31-8.24 (8H), 8.04 (1H), 7.93 (2H), 7.68-7.53 (9H).

### Example 15

### Synthesis of Compound (60)

9-(Quinoline-3-yl)-3,6-bis(4,4,5,5-tetramethyl-1,3,2-dioxaborolane-2-yl)-9H-car bazole: 9.8 g,
6-chloro-2-phenyl quinoxaline: 9.5 g,
tris(dibenzylideneacetone)dipalladium(0): 0.8 g,
tricyclohexylphosphine: 1.0 g, and
tripotassium phosphate: 11.4 g
were added to a reaction vessel, and the mixture was refluxed and stirred in 1,4-dioxane/H₂O mixed solvent overnight.

After the mixture was allowed to cool, MeOH was added thereto, and the precipitated solid was filtered to obtain a crude product.

The obtained crude product was purified through recrystallization using monochlorobenzene/acetone mixed solvent to obtain Compound (60): 8.6 g (yield: 68.2%).

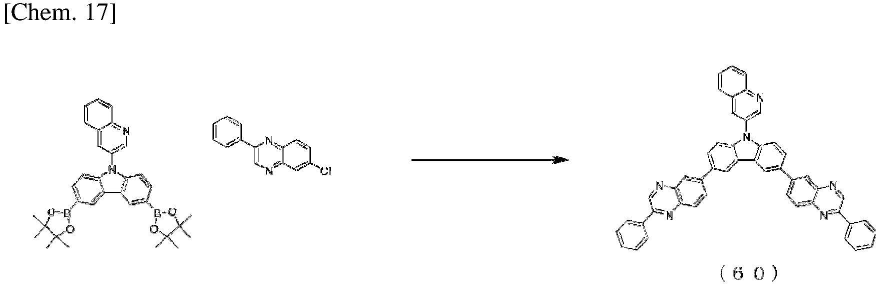

The structure of the obtained yellow powder was identified using NMR.

In ¹H-NMR (CDCl₃), the following signals of 30 hydrogens were detected, and thus it was seen that the obtained material is Compound (60).
δ (ppm) = 9.34 (2H), 9.26 (1H), 8.70 (2H), 8.48 (3H), 8.33-8.24 (9H), 8.01 (1H), 7.95 (2H), 7.89 (1H), 7.74 (1H), 7.63-7.53 (8H).

### Example 16

### Synthesis of Compound (71)

9-(Pyridine-3-yl)-3,6-bis(4,4,5,5-tetramethyl-1,3,2-dioxaborolane-2-yl)-9H-carb azole: 9.0 g,
2-(4-chlorophenyl)-quinoxaline: 2.2 g,
tris(dibenzylideneacetone)dipalladium(0): 0.7 g,
tricyclohexylphosphine: 1.0 g, and
tripotassium phosphate: 11.6 g
were added to a reaction vessel, and the mixture was refluxed and stirred in 1,4-dioxane/H₂O mixed solvent overnight.

After the mixture was allowed to cool, MeOH was added thereto, and the precipitated solid was filtered to obtain a crude product.

The obtained crude product was purified through recrystallization using monochlorobenzene solvent to obtain Compound (71): 5.9 g (yield: 50.0%).

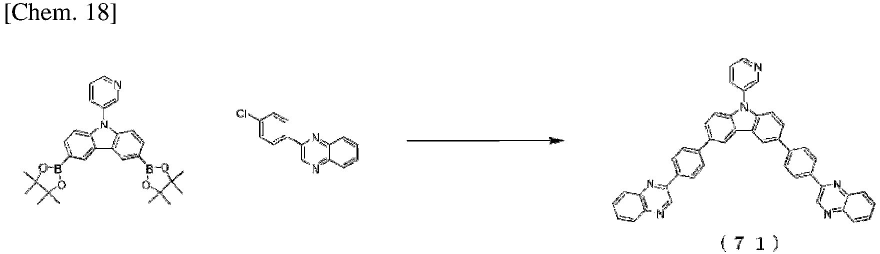

The structure of the obtained yellow powder was identified using NMR.

In ¹H-NMR (CDCl₃), the following signals of 28 hydrogens were detected, and thus it was seen that the obtained material is Compound (71).
δ (ppm) = 7.52 (2H), 7.65 (1H), 7.75-7.84 (6H), 8.00 (5H), 8.18 (4H), 8.36 (4H), 8.55 (2H), 8.80 (1H), 9.00 (1H), 9.43 (2H).

### Example 17

### Synthesis of Compound (98)

3,6-Di(quinoxaline-2-yl)-9H-carbazole: 9.0 g,
2-(4-chlorophenyl)-pyrimidine: 4.5 g,
tris(dibenzylideneacetone)dipalladium(0): 0.4 g,
Sphos: 0.4 g, and
tert-butyl sodium: 4.1 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (98): 7.4 g (yield: 60.1%).

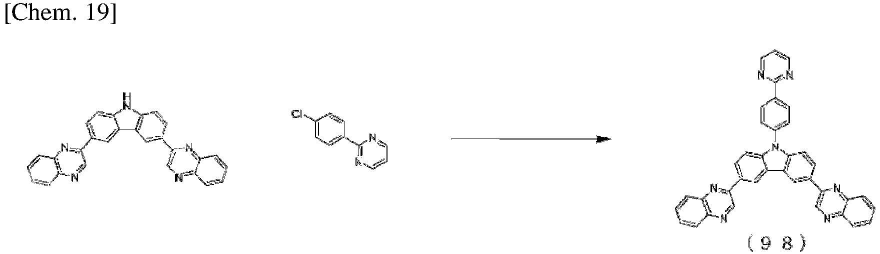

The obtained yellow powder was analyzed using LC-MS, and the same molecular weight was detected.
MS[M+H]⁺= 578

### Example 18

### Synthesis of Compound (99)

3,6-Di(quinoxaline-2-yl)-9H-carbazole: 9.1 g,
2-(3-chlorophenyl)-quinoxaline: 5.7 g,
tris(dibenzylideneacetone)dipalladium(0): 0.4 g,
Sphos: 0.4 g, and
tert-butyl sodium: 4.1 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (99): 6.8 g (yield: 50.4%).

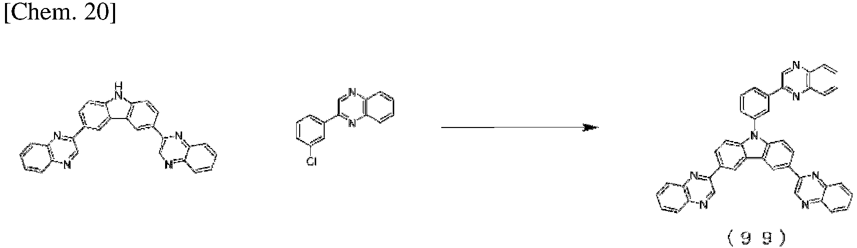

The obtained yellow powder was analyzed using LC-MS, and the same molecular weight was detected.
MS[M+H]⁺= 628

### Example 19

### Synthesis of Compound (100)

3,6-Di(quinoxaline-2-yl)-9H-carbazole: 9.0 g,
2-chloro-quinoxaline: 4.2 g,
tris(dibenzylideneacetone)dipalladium(0): 1.0 g,
Sphos: 1.3 g, and
tert-butyl sodium: 6.1 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (100): 9.6 g (yield: 81.9%).

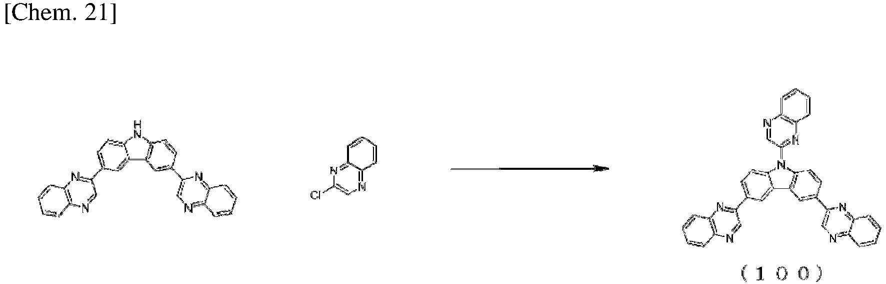

The obtained yellow powder was analyzed using LC-MS, and the same molecular weight was detected.
MS[M+H]⁺= 552

### Example 20

### Synthesis of Compound (101)

3,6-Di(quinoxaline-2-yl)-9H-carbazole: 10.0 g,
2-(3-chlorophenyl)-oxazolopyridine: 7.2 g,
tris(dibenzylideneacetone)dipalladium(0): 0.4 g,
Sphos: 0.4 g, and
tert-butyl sodium: 4.5 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (101): 12.1 g (yield: 84.6%).

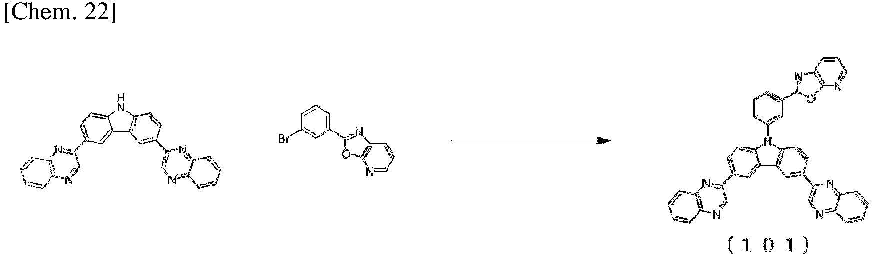

The obtained yellow powder was analyzed using LC-MS, and the same molecular weight was detected.
MS[M+H]⁺= 618

### Example 21

### Synthesis of Compound (102)

3,6-Di(quinoxaline-2-yl)-9H-carbazole: 10.0 g,
3-(4-chlorophenyl)benzofuran: 6.5 g,
tris(dibenzylideneacetone)dipalladium(0): 1.1 g,
Sphos: 1.5 g, and
tert-butyl sodium: 3.4 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (102): 11.4 g (yield: 78.4%).

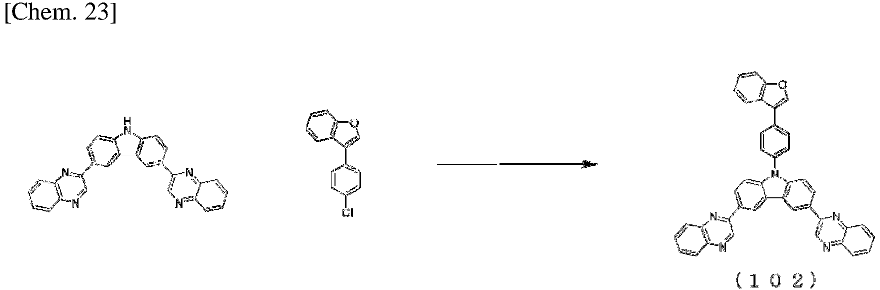

The structure of the obtained yellow powder was identified using NMR.

In ¹H-NMR (CDCl₃), the following signals of 25 hydrogens were detected, and thus it was seen that the obtained material is Compound (102).
δ (ppm) = 9.55 (2H), 9.21 (2H), 8.39 (2H), 8.24 (2H), 8.16 (2H), 7.98 (4H), 7.85-7.75 (6H), 7.69 (2H), 7.63 (1H), 7.42 (2H).

### Example 22

### Synthesis of Compound (103)

2-(4-(6-(Quinoxaline-2-yl)-9H-carbazole-3-yl)phenyl)oxazolopyridine: 5.6 g, 2-chloroquinoline: 2.1 g,
tris(dibenzylideneacetone)dipalladium(0): 0.5 g,
Sphos: 0.5 g, and
tert-butyl sodium: 1.6 g
were added to a reaction vessel, and the mixture was refluxed and stirred in xylene solvent overnight.

After the mixture was allowed to cool, MeOH was added thereto, and the precipitated solid was filtered to obtain a crude product.

The obtained crude product was purified through recrystallization using dichlorobenzene solvent to obtain Compound (103): 4.3 g (yield: 60.1%).

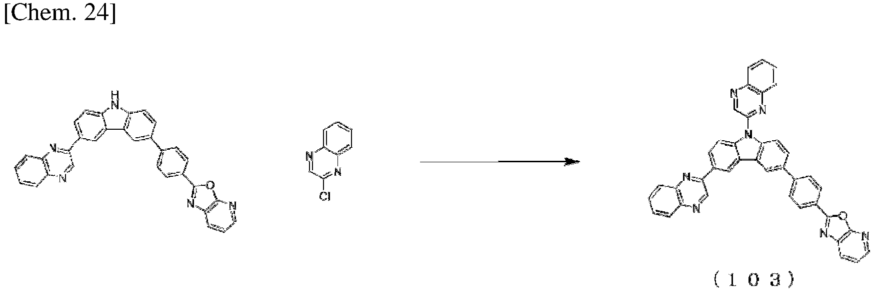

The obtained yellow powder was analyzed using LC-MS, and the same molecular weight was detected.
MS[M+H]⁺= 618

### Example 23

The melting point and the glass transition point (Tg) of each of the compounds obtained in Examples 1 to 22 above were measured using a high-sensitivity differential scanning calorimeter (DSC3100SA manufactured by Bruker AXS K.K.). Table 1 shows the measurement results.

Compounds (18) and (103) exhibited two melting points. This is presumed to be due to the presence of two crystalline forms, each having a different melting point.

**Table 1**

| | Melting point | Glass transition point |
|---|---|---|
| Compound (3) | 333.1 | - |
| Compound (6) | 325.8 | - |
| Compound (10) | 323.4 | - |
| Compound (13) | 266.0 | 119.4 |
| Compound (17) | 295 | 131.7 |
| Compound (18) | 325.3, 319.0 | 133.2 |
| Compound (35) | 327.1 | - |
| Compound (36) | 298.1 | - |
| Compound (37) | 318.5 | - |
| Compound (39) | 362.7 | - |
| Compound (54) | 335.4 | 157.1 |
| Compound (56) | 317.7 | 143.7 |
| Compound (59) | 311.2 | 142.4 |
| Compound (60) | 275.5 | 149.8 |
| Compound (69) | 298.7 | 134.1 |
| Compound (71) | 324.5 | 136.2 |
| Compound (98) | 286.5 | 122 |
| Compound (99) | 284.4 | 123.6 |
| Compound (100) | 308.8 | 135 |
| Compound (101) | 362.5 | - |
| Compound (102) | 262.5 | 117.2 |
| Compound (103) | 325.6, 338.6 | - |

It is seen from the results that the compounds obtained in Examples 1 to 22 have a high melting point and have no glass transition point or a glass transition point of 100°C or higher. This indicates that these compounds are stable in a thin film state and have excellent durability.

### Example 24

A vapor-deposited film with a film thickness of 80 nm was formed on a silicon substrate using each of the compounds obtained in Examples 1 to 22 above, and a refractive index *n* and an extinction coefficient *k* at wavelengths of 450 and 750 nm were measured at room temperature (25±2°C) using a spectroscopic measurement device (F10-RT-UV manufactured by Filmetrics). The refractive index n and the extinction coefficient k of Alq₃ and Comparative Compounds (CPL-1) and (CPL-2) with the structural formulas below were also measured for the sake of comparison (see Patent Literature 4 and Patent Literature 5, for example). Table 2 collectively shows the measurement results.

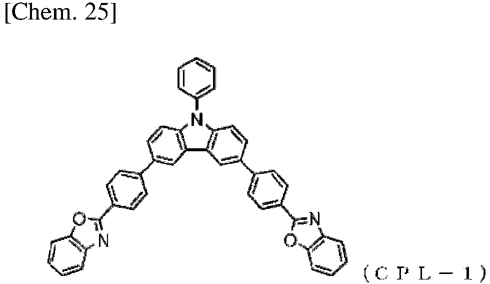

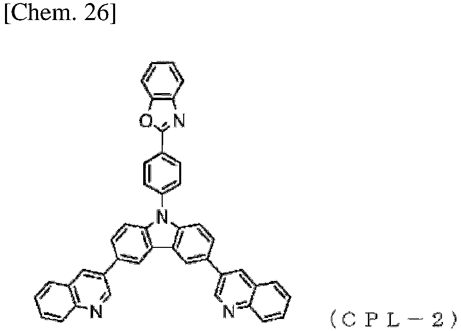

**Table 2**

| | Refractive index n ( 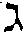 : 450 nm) | Refractive index n ( 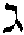 : 750 nm) | Extinction coefficient k ( 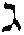 : 450 nm) | Extinction coefficient k ( 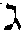 : 750 nm) |
|---|---|---|---|---|
| Compound (3) | 2.30 | 1.96 | 0.00 | 0.00 |
| Compound (6) | 2.34 | 1.98 | 0.00 | 0.00 |
| Compound (10) | 2.36 | 1.98 | 0.01 | 0.00 |
| Compound (13) | 2.33 | 1.96 | 0.01 | 0.00 |
| Compound (17) | 2.33 | 1.97 | 0.00 | 0.00 |
| Compound (18) | 2.46 | 2.00 | 0.03 | 0.00 |
| Compound (35) | 2.36 | 1.98 | 0.00 | 0.00 |
| Compound (36) | 2.36 | 1.97 | 0.00 | 0.00 |
| Compound (37) | 2.37 | 1.98 | 0.00 | 0.00 |
| Compound (39) | 2.37 | 1.99 | 0.01 | 0.00 |
| Compound (54) | 2.44 | 1.98 | 0.03 | 0.00 |
| Compound (56) | 2.47 | 1.98 | 0.09 | 0.00 |
| Compound (59) | 2.45 | 1.98 | 0.04 | 0.00 |
| Compound (60) | 2.45 | 1.98 | 0.06 | 0.00 |
| Compound (69) | 2.40 | 1.98 | 0.02 | 0.00 |
| Compound (71) | 2.36 | 1.97 | 0.00 | 0.00 |
| Compound (98) | 2.43 | 1.99 | 0.01 | 0.00 |
| Compound (99) | 2.48 | 2.00 | 0.03 | 0.00 |
| Compound (100) | 2.41 | 2.00 | 0.03 | 0.00 |
| Compound (101) | 2.41 | 1.99 | 0.01 | 0.00 |
| Compound (102) | 2.31 | 1.96 | 0.00 | 0.00 |
| Compound (103) | 2.49 | 2.01 | 0.00 | 0.00 |
| Alq₃ | 1.88 | 1.73 | 0.03 | 0.00 |
| Compound (CPL-1) | 2.19 | 1.92 | 0.00 | 0.00 |
| Compound (CPL-2) | 2.15 | 1.91 | 0.00 | 0.00 |

As shown in Table 2, at a wavelength of 450 to 750 nm, the carbazole compounds of the present invention have extinction coefficients similar to those of Alq₃ and Comparative Compounds (CPL-1) and (CPL-2), and have refractive indices higher than those thereof. This indicates that an improvement in light extraction efficiency can be expected in an organic EL element in which any of the carbazole compounds of the present invention is used as a constituent material of the capping layer.

### Example 25

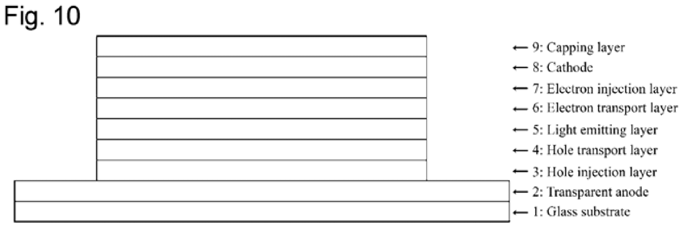

As shown in Fig. 10, an organic EL element was prepared by forming a reflecting ITO electrode serving as a transparent anode 2 on a glass substrate 1 in advance, and furthermore, vapor-depositing a hole injection layer 3, a hole transport layer 4, a light emitting layer 5, an electron transport layer 6, an electron injection layer 7, a cathode 8, and a capping layer 9 in this order on the ITO electrode.

Specifically, a glass substrate 1 on which an ITO film with a thickness of 50 nm, a reflecting film of silver alloy with a film thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was attached inside a vacuum vapor deposition machine, and the pressure was reduced to 0.001 Pa or less. Subsequently, an electron acceptor (Acceptor-1) with the structural formula below and Compound (HTM-1) with the structural formula below were vapor-deposited so as to cover the transparent anode 2 through binary vapor deposition at a vapor deposition rate ratio of (Acceptor-1):Compound (HTM-1) = 3:97, and a film with a thickness of 10 nm was thus formed as a hole injection layer 3.

A film of Compound (HTM-1) with the structural formula below was formed on the hole injection layer 3, as a hole transport layer 4 with a film thickness of 140 nm.

Compound (EMD-1) with the structural formula below and Compound (EMH-1) with the structural formula below were vapor-deposited on the hole transport layer 4 through binary vapor deposition at a vapor deposition rate ratio of (EMD-1):(EMH-1) = 5:95, and a film with a thickness of 20 nm was thus formed as a light emitting layer 5. Compound (ETM-1) with the structural formula below and Compound (ETM-2) with the structural formula below were vapor-deposited on the light emitting layer 5 through binary vapor deposition at a vapor deposition rate ratio of (ETM-1):(ETM-2)= 50:50, and a film with a thickness of 30 nm was thus formed as an electron transport layer 6. A film of lithium fluoride was formed on the electron transport layer 6, as an electron injection layer 7 with a film thickness of 1 nm. A film of magnesium-silver alloy was formed on the electron injection layer 7, as a cathode 8 with a film thickness of 12 nm.

Lastly, a film of Compound (3) of Example 1 was formed as a capping layer 9 with a film thickness of 60 nm. Table 3 collectively shows the measurement results of light emission properties that were obtained when the properties of the produced organic EL element to which a DC voltage was applied were measured in the atmosphere at normal temperature.

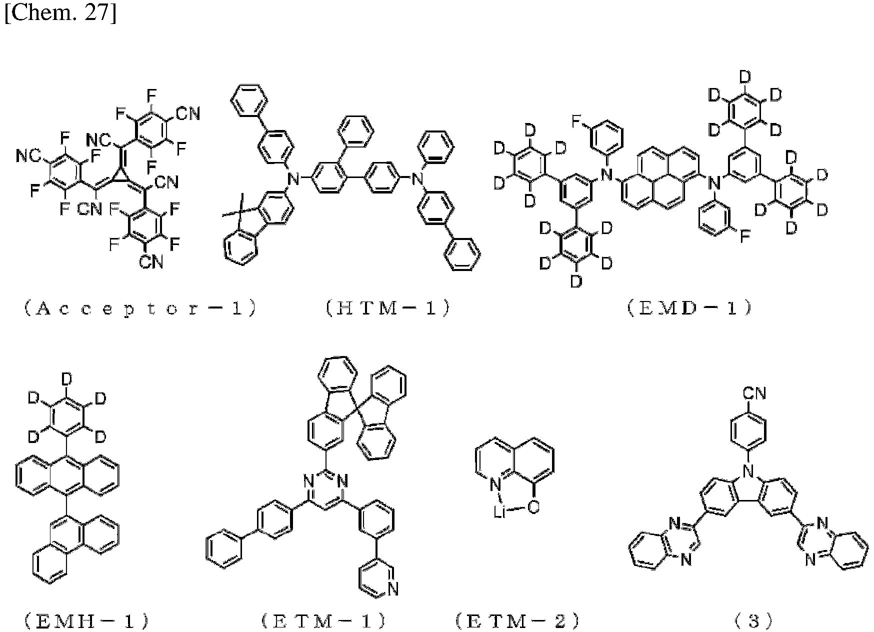

### Example 26

An organic EL element was prepared under similar conditions to those of Example 25, except that Compound (13) obtained in Example 2 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 27

An organic EL element was prepared under similar conditions to those of Example 25, except that Compound (56) obtained in Example 3 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 28

An organic EL element was prepared under similar conditions to those of Example 25, except that Compound (69) obtained in Example 4 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 29

An organic EL element was prepared under similar conditions to those of Example 25, except that Compound (6) obtained in Example 5 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 30

An organic EL element was prepared under similar conditions to those of Example 25, except that Compound (10) obtained in Example 6 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 31

An organic EL element was prepared under similar conditions to those of Example 25, except that Compound (17) obtained in Example 7 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 32

An organic EL element was prepared under similar conditions to those of Example 25, except that Compound (18) obtained in Example 8 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 33

An organic EL element was prepared under similar conditions to those of Example 25, except that Compound (35) obtained in Example 9 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 34

An organic EL element was prepared under similar conditions to those of Example 25, except that Compound (36) obtained in Example 10 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 35

An organic EL element was prepared under similar conditions to those of Example 25, except that Compound (37) obtained in Example 11 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 36

An organic EL element was prepared under similar conditions to those of Example 25, except that Compound (39) obtained in Example 12 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 37

An organic EL element was prepared under similar conditions to those of Example 25, except that Compound (54) obtained in Example 13 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 38

An organic EL element was prepared under similar conditions to those of Example 25, except that Compound (59) obtained in Example 14 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 39

An organic EL element was prepared under similar conditions to those of Example 25, except that Compound (60) obtained in Example 15 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 40

An organic EL element was prepared under similar conditions to those of Example 25, except that Compound (71) obtained in Example 16 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 41

An organic EL element was prepared under similar conditions to those of Example 25, except that Compound (98) obtained in Example 17 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 42

An organic EL element was prepared under similar conditions to those of Example 25, except that Compound (99) obtained in Example 18 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 43

An organic EL element was prepared under similar conditions to those of Example 25, except that Compound (100) obtained in Example 19 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 44

An organic EL element was prepared under similar conditions to those of Example 25, except that Compound (101) obtained in Example 20 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 45

An organic EL element was prepared under similar conditions to those of Example 25, except that Compound (102) obtained in Example 21 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Example 46

An organic EL element was prepared under similar conditions to those of Example 25, except that Compound (103) obtained in Example 22 was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Comparative Example 1

For the sake of comparison, an organic EL element was prepared under similar conditions to those of Example 7, except that Alq₃ was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Comparative Example 2

For the sake of comparison, an organic EL element was prepared under similar conditions to those of Example 7, except that Compound (CPL-1) was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

### Comparative Example 3

For the sake of comparison, an organic EL element was prepared under similar conditions to those of Example 7, except that Compound (CPL-2) was used instead of Compound (3) of Example 1 to form a capping layer 9. Table 3 collectively shows the measurement results of light emission properties that were obtained when the properties of the produced organic EL element to which a DC voltage was applied were measured in the atmosphere at normal temperature.

Table 3 collectively shows the measurement results of element lifespan of the organic EL elements produced in the examples and the comparative examples. The element lifespan as measured in the present invention was defined as follows: when constant current driving was performed at 10 mA/cm² with the initial luminance being set to 100%, the time taken for the luminance to decay to 95% was measured as the element lifespan.

**Table 3**

| | Capping layer | Voltage [V] (@10 mA/cm²) | Luminance [cd/m²] (@10 mA/cm²) | Light emission efficiency [cd/A] (@ 10 mA/cm²) | Power efficiency [lm/W] (@ 10 mA/cm²) | Element lifespan to decay of 95% |
|---|---|---|---|---|---|---|
| Ex. 25 | Compound (3) | 3.64 | 837 | 8.37 | 7.22 | 155 hours |
| Ex. 26 | Compound (13) | 3.63 | 838 | 8.38 | 7.25 | 154 hours |
| Ex. 27 | Compound (56) | 3.62 | 847 | 8.47 | 7.35 | 157 hours |
| Ex. 28 | Compound (69) | 3.63 | 843 | 8.43 | 7.29 | 153 hours |
| Ex. 29 | Compound (6) | 3.64 | 839 | 8.39 | 7.25 | 145 hours |
| Ex. 30 | Compound (10) | 3.65 | 835 | 8.35 | 7.18 | 145 hours |
| Ex. 31 | Compound (17) | 3.63 | 832 | 8.32 | 7.21 | 164 hours |
| Ex. 32 | Compound (18) | 3.62 | 848 | 8.48 | 7.37 | 148 hours |
| Ex. 33 | Compound (35) | 3.65 | 839 | 8.39 | 7.23 | 165 hours |
| Ex. 34 | Compound (36) | 3.64 | 838 | 8.37 | 7.22 | 161 hours |
| Ex. 35 | Compound (37) | 3.64 | 840 | 8.40 | 7.26 | 161 hours |
| Ex. 36 | Compound (39) | 3.63 | 839 | 8.39 | 7.26 | 158 hours |
| Ex. 37 | Compound (54) | 3.62 | 846 | 8.46 | 7.34 | 156 hours |
| Ex. 38 | Compound (59) | 3.61 | 847 | 8.47 | 7.37 | 148 hours |
| Ex. 39 | Compound (60) | 3.61 | 846 | 8.46 | 7.37 | 146 hours |
| Ex. 40 | Compound (71) | 3.63 | 841 | 8.41 | 7.28 | 161 hours |
| Ex. 41 | Compound (98) | 3.63 | 844 | 8.44 | 7.29 | 148 hours |
| Ex. 42 | Compound (99) | 3.63 | 852 | 8.52 | 7.38 | 155 hours |
| Ex. 43 | Compound (100) | 3.63 | 841 | 8.41 | 7.27 | 152 hours |
| Ex. 44 | Compound (101) | 3.61 | 843 | 8.43 | 7.34 | 150 hours |
| Ex. 45 | Compound (102) | 3.64 | 830 | 8.30 | 7.17 | 145 hours |
| Ex. 46 | Compound (103) | 3.65 | 854 | 8.53 | 7.36 | 149 hours |
| Com. Ex. 1 | Alq₃ | 3.65 | 714 | 7.14 | 6.15 | 114 hours |
| Com. Ex. 2 | Compound (CPL-1) | 3.64 | 782 | 7.82 | 6.75 | 141 hours |
| Com. Ex. 3 | Compound (CPL-2) | 3.65 | 824 | 8.24 | 7.07 | 149 hours |

As shown in Table 3, while the elements of Comparative Examples 1 to 3 and those of Examples 25 to 46 had substantially similar driving voltages at a current density of 10 mA/cm² and substantially similar element lifespans, the elements of Examples 25 to 46 exhibited remarkable improvements compared to those of the comparative examples in terms of the luminance, the light emission efficiency, and the power efficiency. This fact means that the carbazole compound represented by the general formula (I) of the present invention is a material that can be favorably used in a capping layer, and can increase the refractive index of the capping layer, thereby being capable of significantly improving the light extraction efficiency of an organic EL element.

### Industrial Applicability

The carbazole compound of the present invention has a high refractive index, can significantly improve the light extraction efficiency, and is stable in a thin film state, and thus it is excellent as a compound that is favorably used in an organic EL element. Furthermore, the organic EL element produced using the carbazole compound of the present invention has high efficiency. Furthermore, the compound of the present invention that does not have absorption in blue, green, and red wavelength regions is particularly favorably used to provide a clear and bright image with good color purity. Therefore, the present invention is expected to be applied to applications such as home electric appliances and lighting equipment, for example.

### List of Reference Numerals

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode
- 9: Capping layer

## Claims

1. A carbazole compound represented by a general formula (I) below: 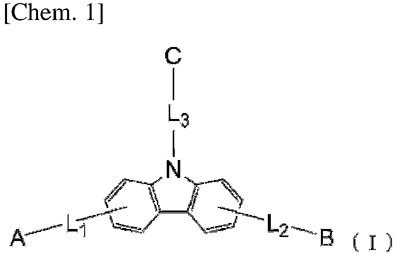
wherein A, B, and C each independently represent a substituted or unsubstituted monovalent aryl group or a substituted or unsubstituted monovalent heteroaryl group,
C is not a benzooxazolyl group, and at least two of A, B, and C represent a substituted or unsubstituted quinoxalyl group or a substituted or unsubstituted quinazolyl group, and
L₁ to L₃ each independently represent a single bond, an unsubstituted divalent aryl group, or an unsubstituted divalent heteroaryl group.

2. The carbazole compound according to claim 1, represented by a general formula (II) below: 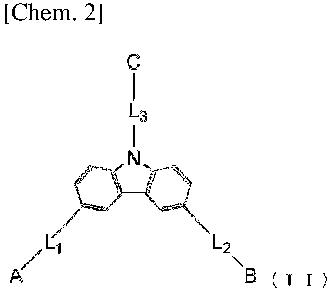 wherein A, B, and C and L₁ to L₃ are as defined in the general formula (I) above.

3. The carbazole compound according to claim 2, wherein in the general formula (II) above, L₁ to L₃ are each independently a single bond, an unsubstituted phenylene group, an unsubstituted pyridylene group, or an unsubstituted naphthylene group.

4. The carbazole compound according to claim 3, wherein in the general formula (II) above, A and B or A and C are each independently a substituted or unsubstituted quinoxalyl group or a substituted or unsubstituted quinazolyl group.

5. The carbazole compound according to claim 4,
wherein in the general formula (II) above, L₁ and L₂ are a single bond, and
A and B are each independently a substituted quinoxalyl group or a substituted quinazolyl group.

6. The carbazole compound according to claim 5, wherein in the general formula (II) above, A and B are each independently a substituted 6-quinoxalyl group or a substituted 6-quinazolyl group.

7. The carbazole compound according to claim 4,
wherein in the general formula (II) above, L₁ and L₂ are a single bond, and
A and B are each independently an unsubstituted quinoxalyl group or an unsubstituted quinazolyl group, or
in the general formula (II) above, L₁ and L₃ are a single bond, and
A and C are each independently an unsubstituted quinoxalyl group or an unsubstituted quinazolyl group.

8. The carbazole compound according to claim 7, wherein in the general formula (II) above, A and B are each independently an unsubstituted 2-quinoxalyl group or an unsubstituted 2-quinazolyl group.

9. The carbazole compound according to claim 4,
wherein in the general formula (II) above, L₁ and L₂ are each independently an unsubstituted phenylene group, an unsubstituted pyridylene group, or an unsubstituted naphthylene group, and
A and B are each independently an unsubstituted quinoxalyl group or an unsubstituted quinazolyl group.

10. The carbazole compound according to claim 7, wherein in the general formula (II) above, A and B are each independently an unsubstituted 2-quinoxalyl group or an unsubstituted 2-quinazolyl group.

11. The carbazole compound according to claim 2, wherein in the general formula (II) above, C is a naphthyl group, a cyanophenyl group, a pyridyl group, a quinolyl group, a quinoxalyl group, a quinazolyl group, a benzothiazolyl group, or an oxazolopyridyl group.

12. The carbazole compound according to claim 11, wherein in the general formula (II) above, C is a 2-naphthyl group, a 4-cyanophenyl group, a 3-pyridyl group, a 4-pyridyl group, a 2-quinolyl group, a 3-quinolyl group, a 6-quinolyl group, a 7-quinolyl group, a 2-quinoxalyl group, a 6-quinoxalyl group, a 2-quinazolyl group, a 6-quinazolyl group, a 7-quinazolyl group, a 2-benzothiazolyl group, or a 2-oxazolopyridyl group.

13. An organic electroluminescent element at least comprising an anode electrode, a hole transport layer, a light emitting layer, an electron transport layer, a cathode electrode, and a capping layer arranged in this order,
wherein the capping layer contains the carbazole compound according to any one of claims 1 to 12.

14. The organic electroluminescent element according to claim 13, wherein a vapor-deposited film, which is obtained by subjecting the carbazole compound according to any one of claims 1 to 12 to vacuum vapor deposition to form a film having a thickness of 80 nm on a silicon substrate, has a refractive index of 1.70 or more at a wavelength of 450 nm or more and 750 nm or less as measured at room temperature (25±2°C).

15. The organic electroluminescent element according to claim 13, wherein the capping layer is constituted by stacked layers or a mixed layer containing two or more types of compounds, and at least one of the compounds is the carbazole compound according to any one of claims 1 to 12.

16. An electronic device or an electronic element comprising a pair of electrodes and at least one organic layer held therebetween, wherein the electronic device or the electronic element contains the carbazole compound according to any one of claims 1 to 12.
